# EUROPEAN PATENT SPECIFICATION

(11) **EP 3 079 654 B1**
(45) Date of publication and mention of the grant of the patent: **14.10.2020**
(21) Application number: 14869848.3
(22) Date of filing: 11.12.2014
(51) Int. Cl.: A61K 8/73, A61Q 5/02, C11D 3/37

(54) **SULFATE-FREE PERSONAL CARE CLEANSING COMPOSITIONS**
SULFATFREIE REINIGUNGSZUSAMMENSETZUNGEN ZUR KÖRPERPFLEGE
COMPOSITIONS DE NETTOYAGE DE SOIN PERSONNEL, EXEMPTES DE SULFATE

(30) Priority: 11.12.2013 US 201361914690 P
(43) Date of publication of application: 19.10.2016
(73) Proprietor: Hercules LLC, Wilmington, DE 19808 (US)
(72) Inventor: DE FEIJ, Eric-Jan, NL-2406 JP Alphen an den Rijn (NL); EVERAERT, Emmanuel Paul Jos Marie, NL-4891 SM Rijsbergen (NL); FRANZKE, Michael Albert Hermann, NL-2993ML Barendrecht (NL); KROON, Gijsbert, NL-3371 BW Hardinxveld Giessendam (NL); NUUTINEN, Tuttu Maria, NL-2613 BT Delft (NL)
(74) Representative: Kutzenberger Wolff & Partner
(86) International application number: PCT/US2014/069684
(87) International publication number: WO 2015/089259

(56) References cited:
- WO-A1-97/26860
- WO-A1-2008/074576
- WO-A2-2013/174615
- US-A- 3 964 500
- US-A- 4 469 627
- US-A- 5 352 387
- US-A1- 2002 077 256
- US-A1- 2003 158 065
- US-A1- 2004 076 595
- US-B2- 6 495 498
- US-B2- 7 947 259
- US-B2- 8 076 279
- US-B2- 8 227 393
- US-B2- 8 546 559

## Description

### BACKGROUND

### 1. FIELD OF THE INVENTION

The presently disclosed and/or claimed inventive process(es), procedure(s), method(s), product(s), result(s), and/or concept(s) (collectively hereinafter referred to as the "presently disclosed and/or claimed inventive concept(s)") relates generally to personal care cleansing compositions such as shampoos and body washes. More particularly, but not by way of limitation, the presently disclosed and/or claimed inventive concept(s) further relates to sulfate-free or substantially sulfate-free personal care cleansing compositions.

### 2. BACKGROUND OF THE INVENTION

Personal care cleansers, such as shampoos, body washes and liquid hand soaps, typically employ sulfate-based surfactant systems (such as, but not limited to, sodium lauryl sulphate and sodium lauryl ether sulfate) because of their effectiveness in foam production and stability, and in deposition of conditioners/health aids to a target substrate such as hair or skin. Such deposition is believed to be via polymer-surfactant complexes, known as coacervates, which are formed upon dilution with water. The conditioners/health aids are entrapped by the coacervates which precipitate out of solution for deposition onto the substrate, thus delivering the conditioners/health aids. Personal care cleansers containing sulfate-based surfactants are also generally easy to thicken with typical thickeners, such as salt and cellulose-based materials.

However, many believe that sulfate-based surfactants are harsh on hair and irritating to skin. In particular, many believe that sulfate-based surfactants cause increased color fading and drying of hair, as well as a drying of the scalp which could lead to dandruff. The main challenges in using sulfate-free surfactants are: 1) difficulty in thickening, 2) poor foaming and foam stability, 3) poor deposition of conditioners/health aids, 4) poor clarity, and 5) poor cleansing. Based on this, the use of sulfate-free surfactants in personal care cleansers typically results in higher costs due to the higher expense of sulfate-free surfactants and the need to use more of such to achieve the same effectiveness as the sulfate-based surfactants.

Thus, there is a need for an improved personal care cleanser which utilizes sulfate-free surfactant(s) and which is economical and more effective, or at least as effective, as sulfate-based cleansers.

### BRIEF DESCRIPTION OF THE FIGURES

FIG. 1A is a plot showing % transparency vs. dilution rate depicting coacervate formation for Formulations A-E and a Commercial Formulation.
FIG. 1B is a plot showing % transparency vs. dilution rate depicting coacervate formation for Formulation F and a Commercial Formulation.
FIG. 2A is a plot showing Instron Wet and Dry Comb total energy test results for hair tresses treated with Formulations A-E and a Commercial Formulation.
FIG. 2B is a plot showing Instron Wet and Dry Comb total energy test results for hair tresses treated with Formulations F and H and a Commercial Formulation.
FIG. 3 is a plot showing foam and liquid height over time for Formulation B.
FIG. 4 is a plot showing foam and liquid height over time for Formulation F and a Commercial Formulation.

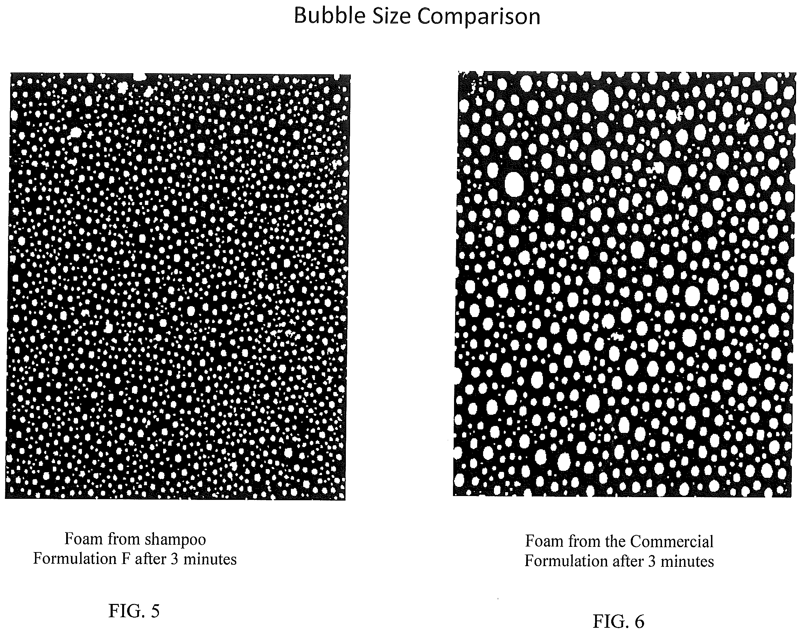

FIG. 5 is a picture showing bubble sizes for a foam created from Formulation F.
FIG. 6 is a picture showing bubble sizes for a foam created from a Commercial Formulation.
FIG. 7 is a plot showing foam and liquid height over time for Formulations I and J.
FIG. 8 is a plot showing foam and liquid height over time for Formulations I and K.
FIG. 9 is a plot showing foam and liquid height over time for Formulations I and L.
FIG. 10 is a plot showing wet state sensory test results for hair tresses treated with aqueous Formulation B and a Commercial Formulation.
FIG. 11 is a plot showing dry state sensory test results for hair tresses treated with aqueous Formulation B and a Commercial Formulation.
FIG. 12 is a plot showing foam and liquid height over time for Formulation M.
FIG. 13 is a plot showing foam and liquid height over time for Formulation T.
FIG. 14 is a plot showing foam and liquid height over time for Formulation V.
FIG. 15 is a plot showing % transparency vs. dilution rate depicting coacervate formation for Formulations I. T, V and M.
FIG. 16 is a plot showing wet combing energy test results for hair tresses treated with Formulations I, K, T, V and M after one wash.

### DETAILED DESCRIPTION OF THE INVENTIVE CONCEPT(S)

Before explaining at least one embodiment of the presently disclosed and/or claimed inventive concept(s) in detail, it is to be understood that the presently disclosed and/or claimed inventive concept(s) is not limited in its application to the details of construction and the arrangement of the components or steps or methodologies set forth in the following description or illustrated in the drawings. The presently disclosed and/or claimed inventive concept(s) is capable of other embodiments or of being practiced or carried out in various ways. Also, it is to be understood that the phraseology and terminology employed herein is for the purpose of description and should not be regarded as limiting.

Unless otherwise defined herein, technical terms used in connection with the presently disclosed and/or claimed inventive concept(s) shall have the meanings that are commonly understood by those of ordinary skill in the art. Further, unless otherwise required by context, singular terms shall include pluralities and plural terms shall include the singular.

All patents, published patent applications, and non-patent publications mentioned in the specification are indicative of the level of skill of those skilled in the art to which the presently disclosed and/or claimed inventive concept(s) pertains.

All of the compositions and/or methods disclosed herein can be made and executed without undue experimentation in light of the present disclosure. While the compositions and methods of the presently disclosed and/or claimed inventive concept(s) have been described in terms of preferred embodiments, it will be apparent to those of ordinary skill in the art that variations may be applied to the compositions and/or methods and in the steps or in the sequence of steps of the method described herein without departing from the concept, spirit and scope of the presently disclosed and/or claimed inventive concept(s). All such similar substitutes and modifications apparent to those skilled in the art are deemed to be within the spirit, scope and concept of the presently disclosed and/or claimed inventive concept(s).

As utilized in accordance with the present disclosure, the following terms, unless otherwise indicated, shall be understood to have the following meanings.

The use of the word "a" or "an" when used in conjunction with the term "comprising" may mean "one," but it is also consistent with the meaning of "one or more," "at least one," and "one or more than one." The use of the term "or" is used to mean "and/or" unless explicitly indicated to refer to alternatives only if the alternatives are mutually exclusive, although the disclosure supports a definition that refers to only alternatives and "and/or." Throughout this application, the term "about" is used to indicate that a value includes the inherent variation of error for the quantifying device, the method being employed to determine the value, or the variation that exists among the study subjects. For example, but not by way of limitation, when the term "about" is utilized, the designated value may vary by plus or minus twelve percent, or eleven percent, or ten percent, or nine percent, or eight percent, or seven percent, or six percent, or five percent, or four percent, or three percent, or two percent, or one percent. The use of the term "at least one" will be understood to include one as well as any quantity more than one, including but not limited to, 1, 2, 3, 4, 5, 10, 15, 20, 30, 40, 50, 100, etc. The term "at least one" may extend up to 100 or 1000 or more depending on the term to which it is attached. In addition, the quantities of 100/1000 are not to be considered limiting as lower or higher limits may also produce satisfactory results. In addition, the use of the term "at least one of X, Y, and Z" will be understood to include X alone, Y alone, and Z alone, as well as any combination of X, Y, and Z. The use of ordinal number terminology (i.e., "first", "second", "third", "fourth", etc.) is solely for the purpose of differentiating between two or more items and, unless otherwise stated, is not meant to imply any sequence or order or importance to one item over another or any order of addition.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "or combinations thereof" as used herein refers to all permutations and combinations of the listed items preceding the term. For example, "A, B, C, or combinations thereof" is intended to include at least one of: A, B, C, AB, AC, BC, or ABC and, if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

In accordance with an embodiment of the presently disclosed inventive concept(s) a personal care cleansing composition is provided comprising:
a) water;
b) up to about 10 wt%, based on the total weight of the personal care cleansing composition, of a surfactant selected from the group consisting of an anionic surfactant, an amphoteric surfactant, a nonionic/anionic surfactant mixture, and combinations thereof;
c) a rheology modifying polymer;
d) a cationic-substituted guar; and
e) a copolymer of acrylamidopropyltrimonium chloride and acrylamide; wherein the personal care cleansing composition is sulfate-free or substantially sulfate-free.

The surfactant comprises, consists of, or consists essentially of any such surfactant which is sulfate-free. Anionic surfactants as used herein, either alone or as part of the nonionic/anionic surfactant mixture, include substances having a negatively charged hydrophobe or that carry a negative charge when the pH is elevated to neutrality or above, such as acylamino acids, and salts thereof, for example, acylglutamates, acyl peptides, sarcosinates, and taurates; carboxylic acids, and salts thereof, for example, alkanolic acids and alkanoates, ester carboxylic acids, and ether carboxylic acids; phosphoric acid ester and salts thereof; sulfonic acids and salts thereof, for example, acyl isethionates, alkylaryl sulfonates, alkyl sulfonates, and sulfosuccinates.

Non-limiting examples of anionic surfactants, used either alone or as part of the nonionic/anionic surfactant mixture, include mono-basic salts of acylglutamates that are slightly acidic in aqueous solution, such as sodium acylglutamate and sodium hydrogenated tallow glutamate; salts of acyl-hydrolyzed protein, such as potassium, palmitoyl hydrolyzed milk protein, sodium cocoyl hydrolyzed soy protein, and TEA-abietoyl hydrolyzed collagen; salts of acyl sarcosinates, such as ammonium myristoyl sarcosine, sodium cocoyl sarcosinate, and TEA-lauroyl sarcosinate; salts of sodium methyl acyltaurates, such as sodium lauroyl taurate and sodium methyl cocoyl taurate; alkanoic acids and alkanoates, such as fatty acids derived from animal and vegetable glycerides that form water-soluble soaps and water-insoluble emulsifying soaps, including sodium stearate, aluminum stearate, and zinc undecylenate; ester carboxylic acids, such as dinonoxynol-9-citrate; salts of acyl lactylates such as calcium stearoyl lactylate and laureth-6 citrate; ethercarboxylic acids derived from ethyoxylated alcohols or phenols having varying lengths of polyoxyethylene chains, such as nonoxynol-8 carboxylic acid, and sodium trideceth-13 carboxylate; mono- and di-esters of phosphoric acid and their salts, such as phospholipids, dilaureth-4-phosphate, DEA-oleth-10 phosphate and triethanolamine lauryl phosphate; salts of acylisethionate, such as sodium cocoyl isethionate; alkylarylbenzene sulfonates, such as alpha-olefin sulfonate (AOS) and alkali metal, alkaline earth metal, and alkanolamine salts thereof, and sodium dodecylbenzene sulfonate; alkyl sulfonates, such as sodium C₁₂-C₁₄ olefin sulfonate, sodium cocomonoglyceride sulfonate, sodium C₁₂-C₁₅ pareth-15 sulfonate, and sodium lauryl sulfoacetate; sulfosuccinates, such as mono- and di-esters of sulfosuccinic acid, salts thereof and alkoxylated alkyl and alkylamido derivatives thereof, such as di-C₄-C₁₀ alkyl sodium sulfosuccinate, disodium laureth sulfosuccinate, disodium oleamido MEA-sulfosuccinate, and disodium C₁₂-C₁₅ pareth sulfosuccinate, and the like.

Particularly, the anionic surfactant, used either alone or as part of the nonionic/anionic surfactant mixture, can comprise, consist of, or consist essentially of a compound selected from the group consisting of an ammonium, alkali or earth alkali salt of: a sulfonate, a sulfosuccinate, a carboxylate, a sarcosinate, an isethionate, a sulfoacetate; and combinations thereof. More particularly, the anionic surfactant, used either alone or as part of the nonionic/anionic surfactant mixture, can comprise, consist of, or consist essentially of a compound selected from the group consisting of sodium alpha-olefin sulfonate, disodium laureth sulfosuccinate, sodium laureth-5 (13) carboxylate, sodium lauroyl sarcosinate, sodium cocoyl isethionate, sodium lauryl sulfoacetate, and combinations thereof.

Amphoteric surfactant(s) as used herein can comprise, consist of, or consist essentially of a compound selected from the group consisting of coco amido propyl betaine, cocoamido hydroxyl sultaine, cocamphoacetate, sodium methyl cocoyl taurate, and combinations thereof.

Nonionic surfactant(s) as used herein can comprise, consist of, or consist essentially of a compound selected from the group consisting of an alkyl glucoside, cocoamide monoethanolamine, cocoamide diethanolamine, a glycerol alkyl ester, polyethylene glycol, and combinations thereof.

The rheology modifying polymer comprises, consists of, or consists essentially of a polymer selected from the group consisting of carboxymethylcellulose, hydroxyethylcellulose, hydroxypropylcellulose, hydroxypropylmethylcellulose, hydroxypropyl-Guar, hydroxymethylhydroxyethylcellulose, and combinations thereof. Particularly, the rheology modifying polymer can comprise hydroxypropylmethylcellulose having a methoxyl content from about 26 to about 32 wt%, or about 28% to about 30 wt%, a hydroxypropyl content from about 6 to about 12 wt%, or about 7 to about 12 wt%, and a viscosity from about 10 to about 16,000 mPas, or from about 40 to about 14,000 mPas. The viscosity can be measured using a viscosimeter, particularly using a 2% solution measured with a Ubbelohde or Brookfield (rotational) viscosimeter.

The cationic-substituted guar can have a degree of cationic substitution of about 0.1 to about 0.4, or about 0.15 to about 0.3, and an average molecular weight from about 500,000 to about 1,800,000, or about 800,000 to about 1,200,000, Dalton.

The cationic-substituted guar can be a guar substituted with at least one cationic moiety selected from compounds having the formula: AB; wherein A, independently, is selected from a linear or branched, substituted or unsubstituted C₁ - C₆ alkyl radical; B, independently, is selected from S⁺R₁R₂X⁻, N⁺R₁R₂R₃X⁻, P⁺R₁R₂R₃X⁻, wherein R₁, R₂, and R₃, independently, are selected from the group consisting of hydrogen and linear and branched C₁ - C₂₄ alkyl, and X⁻ is an anion. Particularly, the member A can comprise a compound selected from the group consisting of 3-halo-2-hydroxypropyl group; 2,3-epoxy propyl group; and combinations thereof.

The at least one cationic moiety can be substituted on a hydroxy group of the guar. Particularly, the cationic-substituted guar can be guar hydroxypropyltrimonium chloride.

The copolymer can have a charge density of about 0.75 to about 3.0, or about 1.2 to about 2.8, or about 1.8 to about 2.4; and an average molecular weight from about 500,000 to about 3,000,000, or about 800,000 to about 2,500,000, or about 1,200,000 to about 2,000,000 Dalton.

The surfactant can be present in an amount from about 6.5 to 10, or up to about 8 wt%, based on the total weight of the personal care cleansing composition.

The rheology modifying polymer is present in an amount ranging from 0.1 to 1.5, or from about 0.2 to about 1.0, or from about 0.3 to about 0.8 wt%, based on the total weight of the personal care cleansing composition.

The cationic-substituted guar can be present in an amount ranging from about 0.05 to about 1.5, or from about 0.1 to about 1, or from about 0.15 to about 0.7 wt%, based on the total weight of the personal care cleansing composition.

The copolymer can be present in an amount ranging from about 0.01 to about 0.25 wt%, or from about 0.03 to about 0.2, or from about 0.05 to about 0.15, based on the total weight of the personal care cleansing composition.

In accordance with an embodiment, the personal care cleansing composition can further comprise a metal halide; wherein the personal care cleansing composition including the metal halide has a higher viscosity as compared to an identical composition not including such metal halide. The metal halide can be selected from the group consisting of NaCl, KCl, NH₄Cl, and combinations thereof.

In accordance with an embodiment, the personal care cleansing composition can have a % transparency which is less than about 50%, or less than about 30%, at a water dilution ranging from about 2.5 to about 5 (volume water: volume personal care cleansing composition), as measured by a spectrophotometer. The measurement can be made utilizing light having a wavelength of about 600 nm.

In accordance with an embodiment, the personal care cleansing composition can have a foam height of at least about 85 mm after 300 seconds, or of at least about 80 mm after 400 seconds.

In accordance with an embodiment, the personal care cleansing composition can include additional cationic polymers such as, synthetic quaternary ammonium polymers, which include, but are not limited to, film-forming polymers and conditioning polymers. Non-limiting examples of synthetic quaternary ammonium polymers include polymers and copolymers of dimethyl diallyl ammonium chloride, such as polyquaternium-4, polyquaternium-6, polyquaternium-7, polyquaternium-22, polyquaternium-10, polyquaternium-11 polyquaternium-15, polyquaternium-16, polyquaternium-24, polyquaternium-28, polyquaternium-32, polyquaternium-33, polyquaternium-35, polyquaternium-37, polyquaternium-39, polyquaternium-44, polyquaternium-55, polyquaternium-56, polyquaternium-67, polyquaternium-68, polyquaternium-69, polyquaternium-70, polyquaternium-71, polyquaternium-72, polyquaternium-73, polyquaternium-74, polyquaternium-75, polyquaternium-76, polyquaternium-83, polyquaternium-84, polyquaternium-85, polyquaternium-86, polyquaternium-87, polyquaternium-88, polyquaternium-89, polyquaternium-91, polyquaternium-98, PEG-2-cocomonium chloride, quaternium-52, and the like.

In accordance with an embodiment, a pH adjusting agent or neutralizer can be added to the personal care cleansing composition as variously described above. Thus, the pH adjusting agent can be utilized in any amount necessary to obtain a desired pH value in the final composition. Non-limiting examples of alkaline pH adjusting agents include alkali metal hydroxides, such as sodium hydroxide, and potassium hydroxide; ammonium hydroxide; organic bases, such as triethanolamine, diisopropylamine, dodecylamine, diisopropanolamine, aminomethyl propanol, cocamine, oleamine, morpholine, triamylamine, triethylamine, tromethamine (2-amino-2-hydroxymethyl)-1,3-propanediol), and tetrakis(hydroxypropyl)ethylenediamine; and alkali metal salts of inorganic acids, such as sodium borate (borax), sodium phosphate, sodium pyrophosphate, and the like, and mixtures thereof. Acidic pH adjusting agents can be organic acids, including amino acids, and inorganic mineral acids. Non-limiting examples of acidic pH adjusting agents include acetic acid, citric acid, fumaric acid, glutamic acid, glycolic acid, hydrochloric acid, lactic acid, nitric acid, phosphoric acid, sulfuric acid, tartaric acid, and the like, and mixtures thereof.

Suitable buffering agents include but are not limited to alkali or alkali earth carbonates, phosphates, bicarbonates, citrates, borates, acetates, acid anhydrides, succinates and the like, such as sodium phosphate, citrate, borate, acetate, bicarbonate, and carbonate.

The pH adjusting agent and/or buffering agent is utilized in any amount necessary to obtain and/or maintain a desired pH value in the composition. In accordance with an embodiment, the personal care cleansing composition as variously described above can contain at least one alkalizing (alkaline pH adjusting agent) or acidifying agent (acidic pH adjusting agent) in amounts from 0.01 to 5 wt. % of the total weight of the composition.

The personal care cleansing composition as variously described above can contain a silicone conditioning agent(s) which are commonly used in rinse off hair conditioner products and in shampoo products, such as the so-called "two-in-one" combination cleansing/conditioning shampoos. The conditioning agent is preferably an insoluble silicone conditioning agent. Typically, the conditioning agent will be mixed in the shampoo composition to form a separate, discontinuous phase of dispersed, insoluble particles (also referred to as droplets). The silicone hair conditioning agent phase can be a silicone fluid and can also comprise other ingredients, such as a silicone resin, to improve silicone fluid deposition efficiency or enhance the glossiness of the hair especially when high refractive index (e.g., above about 1.46) silicone conditioning agents are used. The optional silicone hair conditioning agent phase may comprise volatile silicone, nonvolatile silicone, or combinations thereof. The silicone droplets are typically suspended with an optional suspending agent. The silicone conditioning agent particles may comprise volatile silicone, non-volatile silicone, or combinations thereof. Preferred are non-volatile silicone conditioning agents. If volatile silicones are present, they will typically be incidental to their use as a solvent or carrier for commercially available forms of non-volatile silicone materials ingredients, such as silicone gums and resins. The silicone hair conditioning agents for use in conjunction with the personal care cleansing composition as variously described above can have a viscosity of from about 20 to about 2,000,000 centistokes (1 centistokes equals 1×10⁻⁶ m²/s) in one aspect, from about 1,000 to about 1,800,000 centistokes in another aspect, from about 50,000 to about 1,500,000 in a further aspect, and from about 100,000 to about 1,500,000 centistokes in a still further aspect, as measured at 25° C.

The concentration of the silicone conditioning agent can range from about 0.01% to about 4%, by weight of the composition in which it is included. In another aspect, the amount of silicone conditioning agent ranges from about 0.1% to about 8%, from about 0.1% to about 5% in still another aspect, and from about 0.2% to about 3% by wt. in a further aspect, all based on the total weight of the composition.

In one embodiment, the dispersed silicone conditioning agent particles can have a volume average particle diameter ranging from about 5 µm to about 125 µm. For small particle application to hair, the volume average particle diameters range from about 0.01 µm to about 4 µmin one aspect, from about 0.01 µm to about 2 µm in another aspect, and from about 0.01 µm to about 0.5 µm in still another aspect. For larger particle application to hair, the volume average particle diameters typically range from about 5 µm to about 125 µm in one aspect, from about 10 µm to about 90 µm in another aspect, from about 15 µm to about 70 µm in still another aspect, and from about 20 µm to about 50 µm in a further aspect.

Background material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989). Silicone fluids are generally described as alkylsiloxane polymers. Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609.

Silicone fluids include silicone oils, which are flowable silicone materials having a viscosity, as measured at 25° C. of less than 1,000,000 cSt, and typically range from about 5 cSt to about 1,000,000 cSt. Suitable silicone oils include polyalkyl siloxanes, polyaryl siloxanes, polyalkylaryl siloxanes, polyether siloxane copolymers, and mixtures thereof. Other insoluble, non-volatile silicone fluids having hair conditioning properties may also be used.

Silicone oils include polyalkyl, polyaryl siloxanes, or polyalkylaryl siloxanes which conform to the following formula: 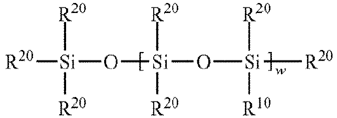 wherein R²⁰ is aliphatic, independently selected from alkyl, alkenyl, and aryl, R²⁰ can be substituted or unsubstituted, and w is an integer from 1 to about 8,000. Suitable unsubstituted R²⁰ groups for use in the personal cleansing compositions described herein include, but are not limited to: alkoxy, aryloxy, alkaryl, arylalkyl, arylalkenyl, alkamino, and ether-substituted, hydroxyl-substituted, and halogen-substituted aliphatic and aryl groups. Suitable R²⁰ groups also include cationic amines and quaternary ammonium groups.

In one embodiment, exemplary R²⁰ alkyl and alkenyl substituents range from C₁-C₅ alkyl and alkenyl, from C₁-C₄ in another aspect, from C₁-C₂ in a further aspect. The aliphatic portions of other alkyl-, alkenyl-, or alkynyl-containing groups (such as alkoxy, alkaryl, and alkamino) can be straight or branched chains, and range from C₁-C₅ in one aspect, from C₁-C₄ in another aspect, and from C₁-C₂ in a further aspect. As discussed above, the R²⁰ substituents can also contain amino functionalities (e.g. alkamino groups), which can be primary, secondary or tertiary amines or quaternary ammonium. These include mono-, di- and tri-alkylamino and alkoxyamino groups, wherein the aliphatic portion chain length is as described above.

Exemplary siloxanes are polydimethyl siloxane, polydiethylsiloxane, and polymethylphenylsiloxane. These siloxanes are available, for example, from the General Electric Company in their Viscasil R and SF 96 series, and from Dow Corning marketed under the Dow Corning 200 series. Exemplary polyalkylaryl siloxane fluids that may be used, include, for example, polymethylphenylsiloxanes. These siloxanes are available, for example, from the General Electric Company as SF 1075 methyl phenyl fluid or from Dow Corning as 556 Cosmetic Grade Fluid.

Cationic silicone fluids are also suitable for use with the personal care cleansing composition as variously described above. The cationic silicone fluids can be represented, but are not limited, to the general formula): (R²¹)ₑG_{3f}-Si-(OSiG₂)_{g}-(OSiG_{f}(R₁)_{(2-f)h}-O-SiG₃₋ₑ(R²¹)_{f} wherein G is hydrogen, phenyl, hydroxy, or C₁-C₈ alkyl, preferably methyl; e is 0 or an integer having from 1 to 3; f is 0 or 1; g is a number from 0 to 1,999; h is an integer from 1 to 2,000, preferably from 1 to 10; the sum of g and h is a number from 1 to 2,000 in one aspect, and from 50 to 500 in another aspect; R²¹ is a monovalent radical conforming to the general formula C_{q}H_{2q}L, wherein q is an integer having a value from 2 to 8 and L is selected from the following groups:
a) -N(R²²)CH₂CH₂N(R²²)₂
b) -N(R²²)
c) -N(R²²)₃CA⁻
d) -N(R²²)CH₂CH₂N(R²²)₂H₂CA⁻
wherein R²² is independently selected from hydrogen, C₁-C₂₀ alkyl, phenyl, benzyl; and A⁻ is a halide ion selected from chloride, bromide, fluoride, and iodide.

An exemplary cationic silicone corresponding to the previous formula defined immediately above is the polymer known as "trimethylsilylamodimethicone" of formula:

(CH₃)₃-Si-[O-Si(CH₃)₂)]_{g}-[O-(CH₃)Si((CH₂)₃-NH-(CH₂)₂-NH₂)]ₕ-O-Si(CH₃)₃

Another cationic silicone useful in combination with the galactomannan substituted compositions as variously described above can be represented by the formula: 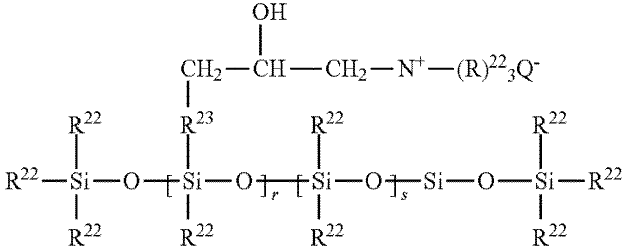 wherein where R²² represents a radical selected from a C₁-C₁₈ alkyl and C₁-C₁₈ alkenyl radical; R²³ independently represents a radical selected from a C₁-C₁₈ alkylene radical or a C₁-C₁₈ alkyleneoxy radical; Q is a halide ion; r denotes an average statistical value from 2 to 20 in one aspect, and from 2 to 8 in another aspect; s denotes an average statistical value from 20 to 200 in one aspect, and from 20 to 50 in another aspect. In one aspect, R²² is methyl. In another aspect, Q is chloride.

Other optional silicone fluids are the insoluble silicone gums. These gums are polysiloxane materials having a viscosity at 25° C. of greater than or equal to 1,000,000 centistokes. Silicone gums are described in U.S. Pat. No. 4,152,416; Noll and Walter, Chemistry and Technology of Silicones, New York: Academic Press 1968; and in General Electric Silicone Rubber Product Data Sheets SE 30, SE 33, SE 54 and SE 76. The silicone gums will typically have a mass molecule weight in excess of about 200,000 Daltons, generally between about 200,000 to about 1,000,000 Daltons, specific examples of which include polydimethylsiloxane, polydimethylsiloxane/methylvinylsiloxane copolymer, polydimethylsiloxane/diphenyl siloxane/methylvinylsiloxane) copolymer, and mixtures thereof.

Another category of nonvolatile, insoluble silicone fluid conditioning agents are the high refractive index polysiloxanes, having a refractive index of at least about 1.46 in one aspect, at least about 1.48 in another aspect, at least about 1.52 in a further aspect, and at least about 1.55 in a still further aspect. The refractive index of the polysiloxane fluid will generally be less than about 1.70, typically less than about 1.60. In this context, polysiloxane "fluid" includes oils as well as gums.

The high refractive index polysiloxane fluid includes those represented by the general formula set forth for the polyalkyl, polyaryl, and polyalkylaryl siloxanes described above, as well as cyclic polysiloxanes (cyclomethicones) represented by the formula: 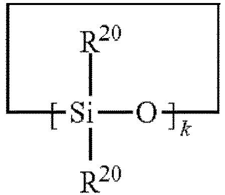 wherein the substituent R²⁰ is as defined above, and the number of repeat units, k, ranges from about 3 to about 7 in one aspect, and from 3 to 5 in another aspect. The high refractive index polysiloxane fluids can contain an amount of aryl containing R²⁰ substituents sufficient to increase the refractive index to the desired level, which is described above. Additionally, R²⁰ and k must be selected so that the material is non-volatile. Aryl containing substituents include those which contain alicyclic and heterocyclic five and six member aryl rings and those which contain fused five or six member rings. The aryl rings can be substituted or unsubstituted. Substituents include aliphatic substituents, and can also include alkoxy substituents, acyl substituents, ketones, halogens (e.g., Cl and Br), amines, etc. Exemplary aryl containing groups include substituted and unsubstituted arenes, such as phenyl, and phenyl derivatives such as phenyls with C₁-C₅ alkyl or alkenyl substituents, e.g., allylphenyl, methyl phenyl and ethyl phenyl, vinyl phenyls such as styrenyl, and phenyl alkynes (e.g. phenyl C₂-C₄ alkynes). Heterocyclic aryl groups include substituents derived from furan, imidazole, pyrrole, pyridine, etc. Fused aryl ring substituents include, for example, naphthalene, coumarin, and purine.

The high refractive index polysiloxane fluids will have a degree of aryl containing substituents of at least about 15% by wt. in one aspect, at least about 20% by wt. in another aspect, at least about 25% by wt. in a further aspect, at least about 35% by wt. in still further aspect, and at least about 50% by wt. in an additional aspect, based on the wt. of the polysiloxane fluid. Typically, the degree of aryl substitution will be less than about 90% by wt., more typically less than about 85% by wt., and can generally ranges from about 55% to about 80% by wt. of the polysiloxane fluid.

In another aspect, the high refractive index polysiloxane fluids have a combination of phenyl or substituted phenyl derivatives. The substituents can be selected from C₁-C₄ alkyl (e.g., methyl), hydroxy, and C₁-C₄ alkylamino (e.g., -R²⁴NHR²⁵NH₂ wherein each R²⁴ and R²⁵ group independently is a C₁-C₃ alkyl, alkenyl, and/or alkoxy.

When high refractive index silicones are used, they optionally can be used in solution with a spreading agent, such as a silicone resin or a surfactant, to reduce the surface tension by a sufficient amount to enhance spreading and thereby enhance the glossiness (subsequent to drying) of hair treated with such compositions. Silicone fluids suitable for use are disclosed in U.S. Pat. No. 2,826,551, U.S. Pat. No. 3,964,500, U.S. Pat. No. 4,364,837, British Pat. No. 849,433, and Silicon Compounds, Petrarch Systems, Inc. (1984). High refractive index polysiloxanes are available from Dow Corning Corporation (Midland, Mich.) Huls America (Piscataway, N.J.), and General Electric Silicones (Waterford, N.Y.).

Silicone resins can be included in the silicone conditioning agent suitable for use in combination with the personal care cleansing composition as variously described above. These resins are crosslinked polysiloxanes. The crosslinking is introduced through the incorporation of trifunctional and tetrafunctional silanes with monofunctional or difunctional (or both) silanes during manufacture of the silicone resin.

As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units (and hence, a sufficient level of crosslinking) such that they dry down to a rigid, or hard, film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein. In one aspect, the ratio of oxygen:silicon atoms is at least about 1.2:1.0. Silanes used in the manufacture of silicone resins include monomethyl-, dimethyl-, trimethyl-, monophenyl-, diphenyl-, methylphenyl-, monovinyl-, and methylvinyl-chlorosilanes, and terachlorosilane, with the methyl-substituted silanes being most commonly utilized. Silicone resins are offered by General Electric as GE SS4230 and SS4267.

Silicone materials and silicone resins in particular, are identified according to a shorthand nomenclature system known to those of ordinary skill in the art as "MDTQ" nomenclature. Under this system, the silicone is described according to the presence of various siloxane monomer units which make up the silicone. Briefly, the symbol M denotes the monofunctional unit (CH₃)₃SiO_{0.5}; D denotes the difunctional unit (CH₃)₂SiO; T denotes the trifunctional unit (CH₃)SiO_{1.5}; and Q denotes the quadra- or tetra-functional unit SiO₂. Primes of the unit symbols (e.g. M', D', T', and Q') denote substituents other than methyl, and must be specifically defined for each occurrence. Typical alternate substituents include groups such as vinyl, phenyls, amines, hydroxyls, etc. The molar ratios of the various units, either in terms of subscripts to the symbol indicating the total number of each type of unit in the silicone (or an average thereof) or as specifically indicated ratios in combination with molecular weight complete the description of the silicone material under the MDTQ system. Higher relative molar amounts of T, Q, T' and/or Q' to D, D', M and/or M' in a silicone resin is indicative of higher levels of crosslinking. As discussed before, however, the overall level of crosslinking can also be indicated by the oxygen to silicon ratio.

Exemplary silicone resins include, but are not limited to MQ, MT, MTQ, MDT and MDTQ resins. In one aspect, methyl is the silicone resin substituent. In another aspect, the silicone resin is selected from a MQ resins, wherein the M:Q ratio is from about 0.5:1.0 to about 1.5:1.0 and the average molecular weight of the silicone resin is from about 1000 to about 10,000 Daltons.

When employed with non-volatile silicone fluids having a refractive index below 1.46, the weight ratio of the non-volatile silicone fluid to the silicone resin component, ranges from about 4:1 to about 400:1 in one aspect, from about 9:1 to about 200:1 in another aspect, from about 19:1 to about 100:1 in a further aspect, particularly when the silicone fluid component is a polydimethylsiloxane fluid or a mixture of polydimethylsiloxane fluid and polydimethylsiloxane gum as described above. Insofar as the silicone resin forms a part of the same phase in the compositions hereof as the silicone fluid, i.e., the conditioning active, the sum of the fluid and resin should be included in determining the level of silicone conditioning agent in the composition.

The volatile silicones described above include cyclic and linear polydimethylsiloxanes, and the like. Cyclic volatile silicones (cyclomethicones) typically contain about 3 to about 7 silicon atoms, alternating with oxygen atoms, in a cyclic ring structure such as described above for the non-volatile cyclic silicones. However, each R²⁰ substituent and repeating unit, k, in the formula must be selected so that the material is non-volatile. Typically, R²⁰ is substituted with two alkyl groups (e.g., methyl groups). The linear volatile silicones are silicone fluids, as described above, having viscosities of not more than about 25 mPa·s. "Volatile" means that the silicone has a measurable vapor pressure, or a vapor pressure of at least 2 mm of Hg at 20° C. Non-volatile silicones have a vapor pressure of less than 2 mm Hg at 20° C. A description of cyclic and linear volatile silicones is found in Todd and Byers, "Volatile Silicone Fluids for Cosmetics", Cosmetics and Toiletries, Vol. 91(1), pp. 27-32 (1976), and in Kasprzak, "Volatile Silicones", Soap/Cosmetics/Chemical Specialities, pp. 40-43 (December 1986).

Exemplary volatile cyclomethicones are D4 cyclomethicone (octamethylcyclotetrasiloxane), D5 cyclomethicone (decamethylcyclopentasiloxane), D6 cyclomethicone, and blends thereof (e.g., D4/D5 and D5/D6). Volatile cyclomethicones and cyclomethicone blends are commercially available from G.E. Silicones as SF1173, SF1202, SF1256, and SF1258, Dow Corning Corporation as Dow Corning ® 244, 245, 246, 345, 1401 and 1501 Fluids. Blends of volatile cyclomethicones and volatile linear dimethicones are also contemplated.

Exemplary volatile linear dimethicones include hexamethyldisiloxane, octamethyltrisiloxane, decamethyltetrasiloxane, dodecamethylpentasiloxane and blends thereof. Volatile linear dimethicones and dimethicone blends are commercially available from Dow Corning Corporation as Dow Corning 200® Fluid (e.g., product designations 0.65 CST, 1 CST, 1.5 CST, and 2 CST) and Dow Corning® 2-1184 Fluid.

Emulsified silicones are also suitable for combination with the personal care cleansing composition as variously described above. Typically, silicone emulsions have an average silicone particle size in the composition of less than 30 µm in one aspect, less than 20 µm in another aspect, and less than 10 µm in a further aspect. In an embodiment, the average silicone particle size of the emulsified silicone in the composition is less than 2 µm, and ideally it ranges from 0.01 to 1 µm. Silicone emulsions having an average silicone particle size of <0.15 micrometers are generally termed micro-emulsions. Particle size may be measured by means of a laser light scattering technique, using a 2600D Particle Sizer from Malvern Instruments. Suitable silicone emulsions for use in conjunction with the personal care cleansing composition as variously described above are also commercially available in a pre-emulsified form. Examples of suitable pre-formed emulsions include emulsions DC2-1766, DC2-1784, DC2-1788, and micro-emulsions DC2-1865 and DC2-1870, all available from Dow Corning. These are all emulsions/micro-emulsions of dimethiconol. Crosslinked silicone gums are also available in a pre-emulsified form, which is advantageous for ease of formulation. An exemplary material is available from Dow Corning as DC X2-1787, which is an emulsion of crosslinked dimethiconol gum. Another exemplary material is available from Dow Corning as DC X2-1391, which is a micro-emulsion of crosslinked dimethiconol gum. Preformed emulsions of amino functional silicone are also available from suppliers of silicone oils such as Dow Corning and General Electric. Particularly suitable are emulsions of amino functional silicone oils with non ionic and/or cationic surfactant. Specific examples include DC929 Cationic Emulsion, DC939 Cationic Emulsion, DC949 Cationic emulsion, and the non-ionic emulsions DC2-7224, DC2-8467, DC2-8177 and DC2-8154 (all available from Dow Corning). Mixtures of any of the above types of silicone may also be used. Specific examples of amino functional silicones suitable are the aminosilicone oils DC2-8220, DC2-8166, DC2-8466, and DC2-8950-114 (all available from Dow Corning), and GE 1149-75, (ex General Electric Silicones). An example of a quaternary silicone polymer useful in conjunction with the personal care cleansing composition as variously described above is the material K3474, available from Goldschmidt, Germany.

Other suitable silicone oils include the dimethicone copolyols, which are linear or branched copolymers of dimethylsiloxane (dimethicone) modified with alkylene oxide units. The alkylene oxide units can be arranged as random or block copolymers. A generally useful class of dimethicone polyols are block copolymers having terminal and/or pendent blocks of polydimethylsiloxane and blocks of polyalkylene oxide, such as blocks of polyethylene oxide, polypropylene oxide, or both. Dimethicone copolyols can be water soluble or insoluble depending on the amount of polyalkylene oxide present in the dimethicone polymer and can be anionic, cationic, or nonionic in character.

The water soluble or water dispersible silicones can also be used in combination with personal care cleansing composition as variously described above. Such water soluble silicones contain suitable anionic functionality, cationic functionality, and/or nonionic functionality to render the silicone water soluble or water dispersible. In one embodiment, the water soluble silicones contain a polysiloxane main chain to which is grafted at least one anionic moiety. The anionic moiety can be grafted to a terminal end of the polysiloxane backbone, or be grafted as a pendant side group, or both. By anionic group is meant any hydrocarbon moiety that contains at least one anionic group or at least one group that can be ionized to an anionic group following neutralization by a base. As discussed previously, the quantity of the hydrocarbon groups of anionic character which are grafted onto the silicone chain are chosen so that the corresponding silicone derivative is water-soluble or water-dispersible after neutralization of the ionizable groups with a base. The anionic silicone derivatives can be selected from existing commercial products or can be synthesized by any means known in the art. The nonionic silicones contain alkylene oxide terminal and/or pendant side chain units (e.g., dimethicone copolyols).

Silicones with anionic groups can be synthesized by reaction between (i) a polysiloxane containing a silinic hydrogen and (ii) a compound containing olefinic unsaturation that also contains an anionic functional group. Exemplary of such a reaction is the hydrosilylation reaction between poly(dimethylsiloxanes) containing a Si-H group(s) and an olefin, CH₂=CHR²⁶, wherein R²⁶ represents a moiety containing an anionic group. The olefin can be monomeric, oligomeric or polymeric. Polysiloxane compounds that contain a pendant reactive thio (-SH) group(s) are also suitable for grafting an unsaturated anionic group containing compound to the poly(siloxane) backbone.

According to one aspect, the anionic monomers containing ethylenic unsaturation are used alone or in combination and are selected from linear or branched, unsaturated carboxylic acids. Exemplary unsaturated carboxylic acids are acrylic acid, methacrylic acid, maleic acid, maleic anhydride, itaconic acid, fumaric acid and crotonic acid. The monomers can optionally be partially or completely neutralized by base to form an alkali, alkaline earth metal, and ammonium salt. Suitable bases include but are not limited to the alkali, alkaline earth (e.g., sodium, potassium, lithium, calcium) and ammonium hydroxides. It will be noted that, similarly, the oligomeric and polymeric graft segments formed from the forgoing monomers can be post-neutralized with a base (sodium hydroxide, aqueous ammonia, etc.) to form a salt. Examples of silicone derivatives which are suitable for use are described in patent applications numbers EP-A-0 582,152 and WO 93/23009. An exemplary class of silicone polymers is the polysiloxanes containing repeat units represented by the following structure: 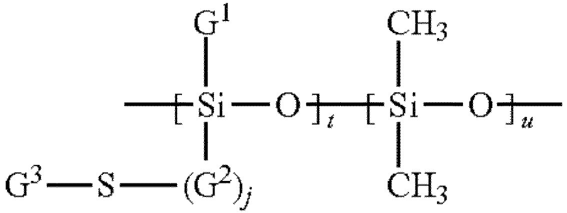 wherein G¹ represents hydrogen, C₁-C₁₀ alkyl and phenyl radical; G² represents C₁-C₁₀ alkylene; G³ represents an anionic polymeric residue obtained from the polymerization of at least one anionic monomer containing ethylenic unsaturation; j is 0 or 1; t is an integer ranging from 1 to 50; and u is an integer from 10 to 350. In an embodiment, G¹ is methyl; j is 1; and G₂ is propylene radical; G³ represents a polymeric radical obtained from the polymerization of at least one unsaturated monomer containing a carboxylic acid group (e.g., acrylic acid, methacrylic acid, itaconic acid, fumaric acid, crotonic acid, maleic acid, or aconitic acid, and the like).

The carboxylate group content in the final polymer preferably ranges from 1 mole of carboxylate per 200 g of polymer to 1 mole of carboxylate per 5000 g of polymer. The number molecular mass of the silicone polymer preferably ranges from 10,000 to 1,000,000 and still more preferably from 10,000 to 100,000. Exemplary unsaturated monomers containing carboxylic acid groups are acrylic acid and methacrylic acid. In addition, to the carboxylic acid group containing monomers, C₁-C₂₀ alkyl esters of acrylic acid and methacrylic acid can be copolymerized into the polymeric backbone. Exemplary esters include but are not limited to the ethyl and butyl esters of acrylic and methacrylic acid. A commercially available silicone-acrylate polymer is marketed by the 3M Company under the trademark Silicones "Plus" Polymer 9857C (VS80 Dry). These polymers contain a polydimethylsiloxanes (PDMS) backbone onto which is grafted (through a thiopropylene group) random repeating units of poly(meth)acrylic acid and the butyl ester of poly(meth)acrylate. These products can be obtained conventionally by radical copolymerization between thiopropyl functionalized polydimethylsiloxane and a mixture of monomers comprising (meth)acrylic acid and of butyl(meth)acrylate.

In another embodiment, the water soluble silicone copolyol can be represented silicone copolyol carboxylates represented by the formula: 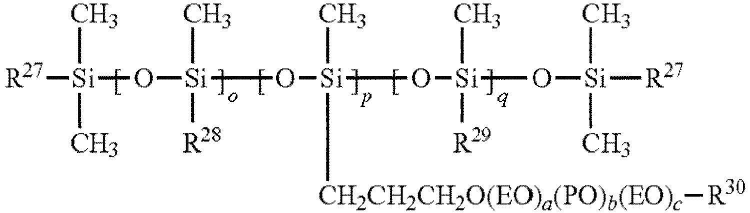 where R²⁷ and R²⁸ are independently selected from C₁-C₃₀ alkyl, C₆-C₁₄ aryl, C₇-C₁₅ aralkyl, C₁-C₁₅ alkaryl, or an alkenyl group of 1 to 40 carbons, hydroxyl, -R³¹-G' or-(CH₂)₃O(EO)ₐ(PO)_{b}(EO)_{c}-G', with the proviso that both R²⁷ and R²⁸ are not methyl; R²⁹ is selected from C₁-C₅ alkyl or phenyl; in this formula a, b, and c are integers independently ranging from 0 to 100; EO is ethylene oxide, -(CH₂CH₂O)-; PO is propylene oxide, -(CH₂CH(CH₃)O)-; in this formula o is an integer ranging from 1 to 200, p is an integer ranging from 0 to 200, and q is an integer ranging from 0 to 1000; R³⁰ is hydrogen, C₁-C₃₀ alkyl, aryl, C₇-C₁₅ aralkyl, C₇-C₁₅ alkaryl, or alkenyl group of 1 to 40 carbons or -C(O)-X wherein X is C₁-C₃₀ alkyl, C₆-C₁₄ aryl, C₇-C₁₅ aralkyl, C₁-C₁₅alkaryl, or an alkenyl group of 1 to 40 carbons, or a mixture thereof; R³¹ is a divalent group selected from alkylene radical of 1 to 40 carbon atoms which may be interrupted with arylene group of 6 to 18 carbons or an alkylene group containing unsaturation of 2 to 8 carbons; and G' is independently are selected from: 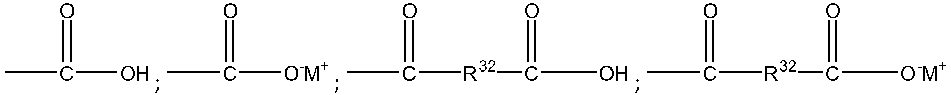 where R³² is a divalent group selected from alkylene of 1 to 40 carbons, an unsaturated group containing 2 to 5 carbon atoms, or an arylene group of 6 to 12 carbon atoms; where M is a cation selected from Na, K, Li, NH₄, or an amine containing C₁-C₁₀ alkyl, C₆-C₁₄ aryl (e.g., phenyl, naphthyl), C₂-C₁₀ alkenyl, C₁-C₁₀ hydroxyalkyl, C₇-C₂₄ arylalkyl or C₇-C₂₄ alkaryl groups. Representative R³² radicals are: -CH₂CH₂-, -CH=CH-, -CH=CHCH₂-, and phenylene.

In another embodiment, the water soluble silicones useful in conjunction with the personal care cleansing composition as variously described above can be represented an anionic silicone copolyol represented by the formula: 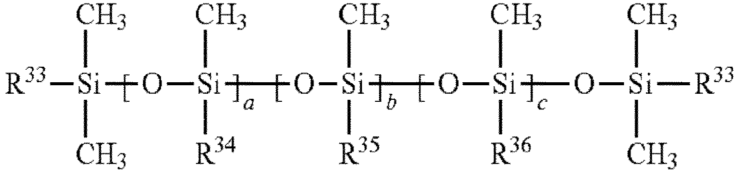 where is R³³ is methyl or hydroxyl; R³⁴ is selected from C₁-C₈ alkyl or phenyl; R³⁵ represents the radical -(CH₂)₃O(EO)ₓ(PO)_{y}(EO)_{z}-SO₃⁻M⁺; where M is a cation selected from Na, K, Li, or NH₄; in this formula x, y and z are integers independently ranging from 0 to 100; R³⁶ represents the radical -(CH₂)₃O(EO)ₓ(PO)_{y}(EO)_{z}-H; in this formula a and c are independently integers ranging from 0 to 50, and b is an integer ranging from 1 to 50; EO is ethylene oxide, e.g.,-(CH₂CH₂O)-; PO is propylene oxide, e.g., -(CH₂CH(CH₃)O)-.

In still another embodiment, the water soluble silicones useful in conjunction with the personal care cleansing composition as variously described above can be represented by an anionic silicone copolyol represented by the formula: 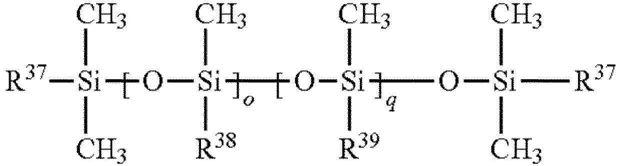 wherein R³⁷ and R³⁸ independently are -CH₃ or a radical represented by:-(CH₂)₃O(EO)ₐ(PO)_{b}(EO)_{c}-C(O)-R⁴⁰-C(O)OH, subject to the proviso that both R³⁷ and R³⁸ are not -CH₃ at the same time; R⁴⁰ is selected from the divalent radical -CH₂CH₂, -CH=CH-, and phenylene; R³⁹ is selected from C₁-C₅ alkyl or phenyl; in this formula a, b and c are integers independently ranging from 0 to 20; EO is an ethylene oxide residue, e.g., -(CH₂CH₂O)-; PO is a propylene oxide residue, e.g., -(CH₂CH(CH₃)O)-; in this formula o is an integer ranging from 1 to 200 and q is an integer ranging from 0 to 500.

Other water soluble silicones useful in conjunction with the personal care cleansing composition as variously described above are quaternized silicone copolyol polymers. These polymers have a pendant quaternary nitrogen functional group present and are represented by the formula: 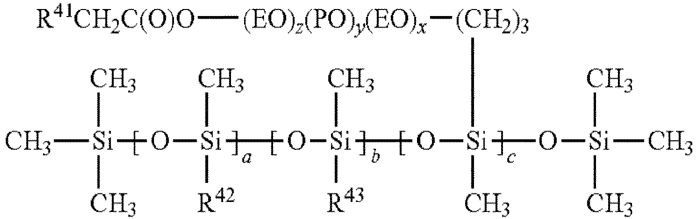 where R⁴¹ represents a quaternary substituent -N⁺R³R⁴R⁵X⁻, wherein R³ and R⁴, and R⁵, independently, are selected from hydrogen and linear and branched C₁-C₂₄ alkyl, and X⁻ represents an anion suitable to balance the cationic charge on the nitrogen atom; R⁴² is selected from C₁-Cₗ₀ alkyl and phenyl; R⁴³ is -(CH₂)₃O(EO)ₓ(PO)_{y}(EO)_{z}-H, where EO is an ethylene oxide residue, e.g., -(CH₂CH₂O)-; PO is a propylene oxide residue, e.g., -(CH₂CH(CH₃)O)-; in this formula a is an integer from 0 to 200, b is an integer from 0 to 200, and c is an integer from 1 to 200; in this formula x, y and z are integers and are independently selected from 0 to 20. In one aspect, the anion X⁻ represents an anion selected from chloride, bromide, iodide, sulfate, methylsulfate, sulfonate, nitrate, phosphate, and acetate.

Other suitable water soluble silicones are amine substituted silicone copolyols represented by the formula: 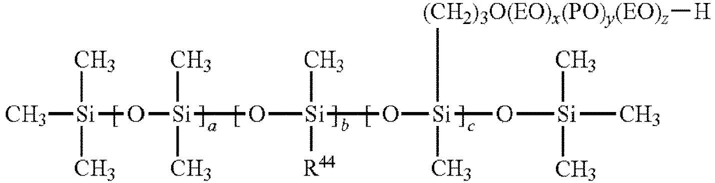 where R⁴⁴ is selected from -NH(CH₂)ₙNH₂ or -(CH₂)ₙNH₂, where in this formula n is an integer from 2 to 6; and x, is n integer from 0 to 20; where EO is an ethylene oxide residue, e.g., - (CH₂CH₂O)-; PO is a propylene oxide residue, e.g., -(CH₂CH(CH₃)O)-; in this formula a is an integer from 0 to 200, b is an integer from 0 to 200, and c is an integer from 1 to 200; in this formula x, y and z are integers and are independently selected from 0 to 20.

Still other water soluble silicones can be selected from nonionic silicone copolyols (dimethicone copolyols) represented by the formula: 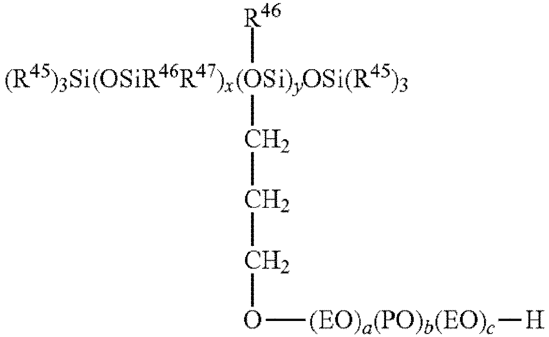 where R⁴⁵, independently, represents a radical selected from C₁-C₃₀ alkyl, C₆-C₁₄ aryl, and C₂-C₂₀ alkenyl; R⁴⁶ represents a radical selected from C₁-C₃₀ alkyl, C₆-C₁₄ aryl, and C₂-C₂₀ alkenyl; EO is an ethylene oxide residue, e.g., -(CH₂CH₂O)-; PO is a propylene oxide residue, e.g.,-(CH₂CH(CH₃)O)-; in this formula a, b, and c are, independently, 0 to 100; in this formula x is 0 to 200; and y is 1 to 200.

In another embodiment, water soluble silicones can be selected from nonionic silicone copolyols represented by the formula: 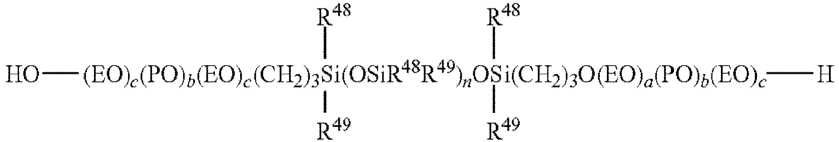 wherein R⁴⁸ and R⁴⁹, independently, represent a radical selected from C₁-C₃₀ alkyl, C₆-C₁₄ aryl, and C₂-C₂₀ alkenyl; EO is an ethylene oxide residue, e.g., -(CH₂CH₂O)-; PO is a propylene oxide residue, e.g., -(CH₂CH(CH₃)O)-; in this formula a, b, and c are independently 0 to 100; and in this formula n is 0 to 200.

In the copolyol embodiments set forth above, the EO and PO residues can be arranged in random, non-random, or blocky sequences.

Dimethicone copolyols are disclosed in U.S. Pat. Nos. 5,136,063 and 5,180,843.

In addition, dimethicone copolyols are commercially available under the Silsofit® and Silwet® brand names from the General Electric Company (GE-OSi). Specific product designations include but are not limited to Silsoft 305, 430, 475, 810, 895, Silwet L 7604 (GE-OSi); Dow Corning® 5103 and 5329 from Dow Corning Corporation; and Abil® dimethicone copolyols, such as, for example WE 09, WS 08, EM 90 and EM 97 from Evonik Goldschmidt Corporation; and Silsense™ dimethicone copolyols, such as Silsense Copolyol-1 and Silsense Copolyol-7, available from Lubrizol Advanced Materials, Inc.

The personal care cleansing composition as variously described above can also comprise from about 0.05% to about 3%, by weight of the composition in one aspect, from about 0.08% to about 1.5% in another aspect, and from about 0.1% to about 1% in a further aspect, of at least one conditioning oil as the conditioning agent, either alone or in combination with other conditioning agents, such as the silicones (described above) and the other conditioning agents described below.

Suitable conditioning oils include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils typically contain about 12 to 19 carbon atoms. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2,2,4,4,6,6,8,8-dimethyl-10-methylundecane and 2,2,4,4,6,6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from BP Chemical Company.

Natural oil conditioners are also useful in conjunction with the personal care cleansing composition as variously described above and include but are not limited to peanut, sesame, avocado, coconut, cocoa butter, almond, safflower, corn, cotton seed, sesame seed, walnut oil, castor, olive, jojoba, palm, palm kernel, argan, cedar, soybean, wheat germ, linseed, sunflower seed; eucalyptus, lavender, vetiver, litsea, cubeba, lemon, sandalwood, rosemary, chamomile, savory, nutmeg, cinnamon, hyssop, caraway, orange, geranium, cade, and bergamot oils, fish oils, glycerol tricaprocaprylate; and mixtures thereof. The natural oils can also be utilized as emollients.

Cationic polymers are also useful as conditioning agents alone or in combination with the other conditioning agents described herein. Suitable cationic polymers can be synthetically derived or modified natural polymers such as the cationically modified polysaccharides. While several of the cationic polymers listed herein as suitable conditioning agents are duplicative of those described above for uses in other applications, those of skill in the art will recognize that many polymers serve multiple functions.

Representative cationic polymer conditioners include but are not limited to homopolymers and copolymers derived from free radically polymerizable acrylic or methacrylic ester or amide monomers. The copolymers can contain one or more units derived from acrylamides, methacrylamides, diacetone acrylamides, acrylic or methacrylic acids or their esters, vinyllactams such as vinyl pyrrolidone or vinyl caprolactam, and vinyl esters. Exemplary polymers include copolymers of acrylamide and dimethyl amino ethyl methacrylate quaternized with dimethyl sulfate or with an alkyl halide; copolymers of acrylamide and methacryloyl oxyethyl trimethyl ammonium chloride; the copolymer of acrylamide and methacryloyl oxyethyl trimethyl ammonium methosulfate; copolymers of vinyl pyrrolidone/dialkylaminoalkyl acrylate or methacrylate, optionally quaternized, such as the products sold under the name GAFQUAT™ by International Specialty Products; the dimethyl amino ethyl methacrylate/vinyl caprolactam/vinyl pyrrolidone terpolymers, such as the product sold under the name GAFFIX™ VC 713 by International Specialty Products; the vinyl pyrrolidone/methacrylamidopropyl dimethylamine copolymer, marketed under the name STYLEZE™ CC 10 available from International Specialty Products; and the vinyl pyrrolidone/quaternized dimethyl amino propyl methacrylamide copolymers such as the product sold under the name GAFQUAT™ HS100 by International Specialty Products.

### EXAMPLES

Table 1 below shows properties for certain Benecel™ type rheology modifiers (commercially available from Ashland Inc.).

**Table 1**

| **Benecel™ type** | **Viscosity range, mPa.s** | **Methoxyl, %** | **Hydroxypropyl, %** | **Gelation Temperature, °C** |
|---|---|---|---|---|
| E50 | 40-60 | 28-30 | 7-12 | ∼58 |
| E4M | 2,700-5,040 | 28-30 | 7-12 | ∼58 |
| E10M | 7,500-14,000 | 28-30 | 7-12 | ∼58 |
| K100M | 75,000-140,000 | 20-24 | 7-12 | 75-85* |

| | | | | |
|---|---|---|---|---|
| * - Estimated | | | | |

### Example 1

Tables 2 - 4 below show the compositions for sulfate-free Formulations A-L which were prepared for testing as described below. Formulations A-E each contained greater than 10 wt% surfactants, and Formulations F-L each contained less than 10 wt% surfactants.

**Table 2**

| **Component** | **Function** | **Formulation** | | | |
|---|---|---|---|---|---|
| | | **A** | **B** | **C** | **D** |
| Deionized Water | | Qs to 100% | Qs to 100% | Qs to 100% | Qs to 100% |
| Disodium EDTA¹ | Chelating agent | 0.15 | 0.15 | 0.15 | 0.15 |
| Guar hydroxypropyl trimonium chloride (and) Acrylamidopropyltrimonium chloride / Acrylamide copolymer² | Conditioner | 0.15 | 0.15 | 0.15 | 0.15 |
| Hydroxypropyl cellulose³ | Rheology modifier | 1.0 | 1.0 | -- | -- |
| Cetyl Hydroxyethyl cellulose | Rheology modifier | -- | -- | 1.2 | 1.2 |
| Glycol Distearate⁴ | Pearlizer | 1.5 | 1.5 | 1.5 | 1.5 |
| Disodium Laureth Sulfosuccinate | Co-surfactant | 8 | 3 | -- | -- |
| Sodium Lauroyl Sarcosinate | Co-surfactant | 3 | -- | -- | -- |
| Ammonium Cocoyl Isethionate | Co-surfactant | -- | 6 | -- | -- |
| Sodium Lauryl Sulfoacetate | Co-surfactant | -- | 3 | -- | -- |
| Sodium C14-16 Olefin Sulfonate | Co-surfactant | -- | -- | 10 | -- |
| Decyl Glucoside | Co-surfactant | -- | -- | 2 | 5 |
| Sodium Laureth-11 Carboxylate | Co-surfactant | -- | -- | -- | 6 |
| Cocamidopropyl Betaine⁵ | Co-surfactant | 4 | 3 | 3 | 4 |
| Methylisothiazolinone (and) Phenylpropanol (and) Propylene Glycol⁶ | Preservative | 0.3 | 0.3 | 0.3 | 0.3 |
| Sodium Hydroxide (33%) | pH regulator | 0.01 | 0.01 | 0.01 | 0.01 |
| Sodium Chloride | Visc regulator | 1 | 1 | 1 | 1 |

| | | | | | |
|---|---|---|---|---|---|
| 1- Dissolvine® Na, commercially available from AkzoNobel Corporate. 2- N-Hance™ 4572, commercially available from Ashland Inc. 3- Klucel™ H, commercially available from Ashland Inc. 4- Tegin® G 1100, commercially available from Evonik Industries. 5- Tego® Betain F KH 5, commercially available from Evonik Industries. 6- Optiphen™ MIT Ultra, commercially available from Ashland Inc. | | | | | |

**Table 3**

| **Component** | **Function** | **Formulation** | | | |
|---|---|---|---|---|---|
| | | **E** | **F** | **G** | **H** |
| Deionized Water | | Qs to 100% | Qs to 100% | Qs to 100% | Qs to 100% |
| Disodium EDTA | Chelating agent | 0.1 | 0.1 | 0.1 | 0.1 |
| Guar hydroxypropyl trimonium chloride (and) Acrylamidopropyltrimonium chloride / Acrylamide copolymer | Conditioner | 0.15 | 0.15 | 0.15 | 0.15 |
| Hydroxypropyl cellulose | Rheology modifier | 1.0 | -- | -- | -- |
| Hydroxypropyl Methylcellulose⁷ | Rheology modifier | -- | 0.6 | -- | 0.6 |
| Glycol Distearate | Pearlizer | 1.5 | 1.5 | 1.5 | 1.5 |
| Disodium Laureth Sulfosuccinate | Co-surfactant | 5.7 | 1.3 | 1.3 | -- |
| Sodium cocoamphoacetate | Co-surfactant | 3.6 | -- | -- | -- |
| Sodium Cocoyl Isethionate⁸ | Co-surfactant | -- | 1.3 | 1.3 | 1.3 |
| Sodium Lauroyl Methyl Isethionate⁹ | Co-surfactant | -- | 4.1 | 4.1 | 4.1 |
| Sodium Methyl Cocoyl Taurate¹⁰ | Co-surfactant | -- | -- | -- | 0.5 |
| Sodium Lauryl Sulfoacetate | Co-surfactant | -- | 1.2 | 1.2 | -- |
| Cocamidopropyl Betaine | Co-surfactant | 5.4 | 1.8 | 1.8 | 2.7 |
| Methylisothiazolinone (and) Phenylpropanol (and) Propylene Glycol | Preservative | 0.2 | 0.2 | 0.2 | 0.2 |
| Sodium Hydroxide (33%) pH | pH regulator | 0.01 | 0.01 | 0.01 | 0.01 |

| | | | | | |
|---|---|---|---|---|---|
| 7- Benecel™ E10, commercially available from Ashland Inc. 8- Hostapon® SCI 185, commercially available from Clariant International Ltd. 9- Iselux® flakes, commercially available from Innospec Performance Chemicals. 10- Pureact® WS Conc., commercially available from Innospec Performance Chemicals. | | | | | |

**Table 4**

| **Trade Name** | **Component** | **Formulation** | | | |
|---|---|---|---|---|---|
| | | **I** | **J** | **K** | **L** |
| | Deionized Water | QS to 100% | QS to 100% | QS to 100% | QS to 100% |

| **Surfactants** | | | | | |
|---|---|---|---|---|---|
| Hostapon® SC185C | Sodium Cocoyl Isethionate | 1.3 | 1.3 | 1.3 | 1.3 |
| Iselux® flakes | Sodium laurovl Methyl Isethionate | 4 | 4 | 4 | 4 |
| Lumerol K5019¹¹ | Disodium Laureth Sulfosuccinate/ Sodium Laurvl Sulfoacetate | 2.4 | 2.4 | 2.4 | 2.4 |
| Tego® Betain F KH 5 | Cocamidopropyl Betaine | 1.8 | 1.8 | 1.8 | 1.8 |

| **Rheology Modifiers** | | | | | |
|---|---|---|---|---|---|
| Benecel™ E10M | Hydroxypropyl Methylcellulose | 0.5 | -- | -- | 0.5 |
| Benecel™ K100M¹² | Hydroxypropyl Methylcellulose | -- | -- | 0.5 | -- |

| **Conditioners** | | | | | |
|---|---|---|---|---|---|
| N-Hance™ 4572 | Guar hydroxypropyltrimonium Chloride (and) Acrylamidopropyltrimonium Chloride/Acrylamide Copolymer | 0.15 | 0.15 | 0.15 | -- |

| **Others** | | | | | |
|---|---|---|---|---|---|
| Tegin® G 1100 | Glycol Distearate | 1.5 | 1.5 | 1.5 | 1.5 |
| Optiphen™ MIT Ultra | Methylisothiazolinone / Phenylpropanol / Propylene Glycol | 0.3 | 0.3 | 0.3 | 0.3 |
| Sodium Hydroxide | Sodium Hydroxide (33%) | 0.01 | 0.01 | 0.01 | 0.01 |
| Dissolvine® Na | Disodium EDTA | 0.15 | 0.15 | 0.15 | 0.15 |
| Sodium Chloride | Sodium Chloride | 1 | 1 | 1 | 1 |
| | Total | 100 | 100 | 100 | 100 |

| | | | | | |
|---|---|---|---|---|---|
| 11 -Lumerol K5019, commercially available from Aschimmer & Schwarz. 12- Benecel™ K100M, commercially available from Ashland Inc. | | | | | |

The physical properties of Formulations I, J, K and L were measured and listed in Table 5. The pH and viscosity values of each Formulation were measured immediately after the formation of the Formulation, one month at 45°C and three month at 45°C.

**Table 5**

| **Properties** | **Measurement** | **Formulation** | | | |
|---|---|---|---|---|---|
| | | **I** | **J** | **K** | **L** |
| **Immediate Measurement** | | | | | |
| pH | | 5.83 | 5.98 | 6.01 | 6.11 |
| Viscosity | Brookfield LVT spindle 63, SP.R/25°C | 5513 | 2021 | 5814 | 5123 |
| **Stability Measurement 1** | 1 month at 45°C | No separation | Separation | No separation | Separation |
| pH | | 5.97 | -- | 5.73 | -- |
| Viscosity | Brookfield LVT spindle 63, SP.R/25°C | 5627 | -- | 5627 | -- |
| **Stability Measurement 2** | 3 month at 45°C | No separation | -- | No separation | -- |
| pH | | 5.73 | - | 6.08 | - |
| Viscosity | Brookfield LVT spindle 63, SP.R/25°C | 5547 | -- | 5987 | -- |

A sulfate-containing commercial shampoo, referred to as "Commercial Formulation" was also used as a control. The Commercial Formulation contained:
43 wt% - Sodium Lauryl Ether Sulfate
6.7 wt% - Cocamidopropyl Betaine
0.15 wt% - Guar hydroxypropyl trimonium chloride (and)
0.16 wt% - Acrylamidopropyltrimonium chloride / Acrylamide copolymer2
0.3 wt% - Parfum
0.3 wt% - Methylisothiazolinone (and) Phenylpropanol (and) Propylene Glycol
0.01 wt% - Sodium Hydroxide
0.15 wt% - Disodium EDTA
1 wt% - Sodium Chloride
Qs to -Deionized Water
100 wt%

Typically, the shampoo formulation is a viscous liquid with pH of about 5.6-6.5 and viscosity of about 4000-6000 mPa.s.

### Example 1A

Table 6 below shows the compositions for sulfate-free formulations M, T and V which were prepared for testing as described below.

The physical properties of Formulations M, T and V were measured and listed in Table 7. The pH and viscosity values of each Formulation were measured immediately after the formation of the Formulation.

**Table 6**

| **Trade Name** | **Component** | **Formulation** | | |
|---|---|---|---|---|
| | | **M** | **T** | **V** |
| | Deionized Water | QS to 100% | QS to 100% | QS to 100% |

| **Surfactants** | | | | |
|---|---|---|---|---|
| Hostapon® SC185C | Sodium Cocoyl Isethionate | 1.3 | 1.3 | 1.3 |
| Iselux® flakes | Sodium lauroyl Methyl | 4 | 4 | 4 |
| Lumerol K5019 | Disodium Laureth Sulfosuccinate/ Sodium Lauryl Sulfoacetate | 2.4 | 2.4 | 2.4 |
| Tego® Betain F KH 5 | Cocamidopropyl Betaine | 1.8 | 1.8 | 1.8 |

| **Rheology Modifiers** | | | | |
|---|---|---|---|---|
| Benecel™ E10M | Hydroxypropyl Methylcellulose | -- | 0.5 | 0.5 |

| **Conditioners** | | | | |
|---|---|---|---|---|
| N-Hance™ 3215¹³ | Guar hydroxypropyltrimonium | -- | 0.2 | -- |
| N-Hance™ CCG45¹⁴ | Guar hydroxypropyltrimonium | -- | -- | 0.2 |

| **Others** | | | | |
|---|---|---|---|---|
| Tegin® G 1100 | Glycol Distearate | -- | 1.5 | 1.5 |
| Firmenich | Parfum | 0.3 | 0.3 | 0.3 |
| Optiphen™ MIT Ultra | Methylisothiazolinone / Phenylpropanol / Propylene Glycol | 0.3 | 0.3 | 0.3 |
| Sodium Hydroxide | Sodium Hydroxide (33%) | 0.01 | 0.01 | 0.01 |
| Dissolvine® Na | Disodium EDTA | 0.15 | 0.15 | 0.15 |
| Sodium Chloride | Sodium Chloride | 1 | 1 | 1 |
| | Total | 100 | 100 | 100 |

| | | | | |
|---|---|---|---|---|
| 13- N-Hance™ 3215, commercially available from Ashland Inc. 14- N-Hance™ CCG45, commercially available from Ashland Inc. | | | | |

**Table 7**

| **Properties** | **Measurement** | **Formulation** | | |
|---|---|---|---|---|
| | | **M** | **T** | **V** |
| **Immediate Measurement** | | | | |
| pH | | 6.15 | 6.23 | 6.00 |
| Viscosity | Brookfield LVT spindle 63, SP.R/25°C | 6529 | 5019 | 899.9 |

### Example 1B

Tables 8-11 below show the composition for sulfate-free formulations N-S, U, W-AJ which are prepared for testing as described below.

**Table 8**

| **Trade Name** | **Component** | **Formulation** | | | | |
|---|---|---|---|---|---|---|
| | | **N** | **O** | **P** | **Q** | **R** |
| | Deionized Water | QS to 100% | QS to 100% | QS to 100% | QS to 100% | QS to 100% |

| **Surfactants** | | | | | | |
|---|---|---|---|---|---|---|
| Hostapon® SC185C | Sodium Cocoyl Isethionate | 1.3 | 1.3 | 1.3 | 1.3 | 1.3 |
| Iselux® flakes | Sodium lauroyl Methyl | 4 | 4 | 4 | 4 | 4 |
| Lumerol K5019 | Disodium Laureth Sulfosuccinate/ Sodium Lauryl Sulfoacetate | 2.4 | 2.4 | 2.4 | 2.4 | 2.4 |
| Tego® Betain F KH | Cocamidopropyl Betaine | 1.8 | 1.8 | 1.8 | 1.8 | 1.8 |

| **Rheology Modifiers** | | | | | | |
|---|---|---|---|---|---|---|
| Benecel™ E10M | Hydroxypropyl | -- | 0.5 | -- | -- | -- |
| Benecel™ E15¹⁵ | Hydroxypropyl | -- | -- | 0.5 | -- | -- |
| Benecel™ K100 | Hydroxypropyl | -- | -- | -- | 0.5 | -- |

| **Conditioners** | | | | | | |
|---|---|---|---|---|---|---|
| N-Hance™ 4572 | Guar hydroxypropyltrimonium Chloride (and) Acrylamidopropyltrimonium Chloride/Acrylamide Copolymer | -- | -- | -- | | 0.15 |

| **Others** | | | | | | |
|---|---|---|---|---|---|---|
| Tegin® G 1100 | Glycol Distearate | 1.5 | 1.5 | 1.5 | 1.5 | - |
| Firmenich | Parfum | 0.3 | 0.3 | 0.3 | 0.3 | 0.3 |
| Optiphen™ MIT Ultra | Methylisothiazolinone / Phenylpropanol / Propylene Glycol | 0.3 | 0.3 | 0.3 | 0.3 | 0.3 |
| Sodium Hydroxide | Sodium Hydroxide (33%) | 0.01 | 0.01 | 0.01 | 0.01 | 0.01 |
| Dissolvine® Na | Disodium EDTA | 0.15 | 0.15 | 0.15 | 0.15 | 0.15 |
| Sodium Chloride | Sodium Chloride | 1 | 1 | 1 | 1 | 1 |
| | Total | 100 | 100 | 100 | 100 | 100 |

| | | | | | | |
|---|---|---|---|---|---|---|
| 15- Benecel™ E15, commercially available from Ashland Inc. | | | | | | |

**Table 9**

| **Trade Name** | **Component** | **Formulation** | | | |
|---|---|---|---|---|---|
| | | **S** | **U** | **W** | **X** |
| | Deionized Water | QS to 100% | QS to 100% | QS to 100% | QS to 100% |

| **Surfactants** | | | | | |
|---|---|---|---|---|---|
| Hostapon® SC185C | Sodium Cocoyl Isethionate | 1.3 | 1.3 | 1.3 | 1.3 |
| Iselux® flakes | Sodium laurovl Methyl | 4 | 4 | 4 | 4 |
| Lumerol K5019 | Disodium Laureth Sulfosuccinate/ Sodium Lauryl Sulfoacetate | 2.4 | 2.4 | 2.4 | 2.4 |
| Tego® Betain F KH 5 | Cocamidopropyl Betaine | 1.8 | 1.8 | 1.8 | 1.8 |

| **Rheology Modifiers** | | | | | |
|---|---|---|---|---|---|
| Benecel™ E10M | Hydroxypropyl | 0.5 | 0.5 | 0.5 | 0.5 |

| **Conditioners** | | | | | |
|---|---|---|---|---|---|
| N-Hance™ 4572 | Guar hydroxypropyltrimonium Chloride (and) Acrylamidopropyltrimonimn Chloride/Acrylamide Copolymer | 0.15 | -- | 0.15 | -- |
| N-Hance™ CG 13¹⁶ | Guar | -- | 0.2 | -- | -- |
| N-Hance™ CCG45¹⁷ | Guar | -- | -- | -- | -- |
| N-Hance™ 5182D¹⁸ | Acrylamidopropyltrimonium | -- | -- | -- | 0.2 |

| **Others** | | | | | |
|---|---|---|---|---|---|
| Dow Corning® 1788¹⁹ | Silicone | -- | -- | 3 | -- |
| Tegin® G 1100 | Glycol Distearate | 1.5 | 1.5 | 1.5 | 1.5 |
| Firmenich | Parfum | 0.3 | 0.3 | 0.3 | 0.3 |
| Optiphen™ MIT Ultra | Methylisothiazolinone / Phenylpropanol / Propylene Glycol | 0.3 | 0.3 | 0.3 | 0.3 |
| Sodium Hydroxide | Sodium Hydroxide (33%) | 0.01 | 0.01 | 0.01 | 0.01 |
| Dissolvine® Na | Disodium EDTA | 0.15 | 0.15 | 0.15 | 0.15 |
| Sodium Chloride | Sodium Chloride | 1 | 1 | 1 | 1 |
| | Total | 100 | 100 | 100 | 100 |

| | | | | | |
|---|---|---|---|---|---|
| 16- N-Hance™ CG 13, commercially available from Ashland Inc. 17- N-Hance™ CCG45, commercially available from Ashland Inc. 18- N-Hance™ 5182D, commercially available from Ashland Inc. 19- Dow Corning® 1788, commercially available from Dow Corning Corporation. | | | | | |

**Table 10**

| **Trade Name** | **Component** | **Formulation** | | | | | |
|---|---|---|---|---|---|---|---|
| | | **Y** | **Z** | **AA** | **AB** | **AC** | **AD** |
| | Deionized Water | QS to 100% | QS to 100% | QS to 100% | QS to 100% | QS to 100% | QS to 100% |

| **Surfactants** | | | | | | | |
|---|---|---|---|---|---|---|---|
| Hostapon® SC185C | Sodium Cocoyl Isethionate | 2 | 2 | 2 | 2 | 2 | 2 |
| Iselux® flakes | Sodium laurovl Methyl | 3.5 | 3.5 | 3.5 | 3.5 | 3.5 | 3.5 |
| Tego® Betain F KH 5 | Cocamidopropyl Betaine | 2.7 | 2.7 | 2.7 | 2.7 | 2.7 | 2.7 |
| Pureact W conc²⁰ | Sodium Methyl Cocoyl Taurate | 1.5 | 1.5 | 1.5 | 1.5 | 1.5 | 1.5 |
| **Rheology Modifiers** | | | | | | | |
| Benecel™ E10M | Hydroxypropyl Methylcellulose | -- | -- | 0.5 | -- | -- | -- |
| Benecel™ E15 | Hydroxypropyl Methylcellulose | -- | -- | -- | 0.5 | -- | -- |
| Benecel™ K100 | Hydroxypropyl Methylcellulose | -- | -- | -- | -- | 0.5 | -- |

| **Conditioners** | | | | | | | |
|---|---|---|---|---|---|---|---|
| N-Hance™ 4572 | Guar hydroxypropyltrimonium Chloride (and) Acrylamidopropyltrimonium Chloride/Acrylamide Copolymer | -- | -- | -- | -- | -- | 0.15 |

| **Others** | | | | | | | |
|---|---|---|---|---|---|---|---|
| Tegin® G 1100 | Glycol Distearate | -- | 1.5 | 1.5 | 1.5 | 1.5 | - |
| Firmenich | Parfum | 0.3 | 0.3 | 0.3 | 0.3 | 0.3 | 0.3 |
| Optiphen™ MIT Ultra | Methylisothiazolinone / Phenylpropanol / Propylene Glycol | 0.3 | 0.3 | 0.3 | 0.3 | 0.3 | 0.3 |
| Sodium Hydroxide | Sodium Hydroxide (33%) | 0.01 | 0.01 | 0.01 | 0.01 | 0.01 | 0.01 |
| Dissolvine® Na | Disodium EDTA | 0.15 | 0.15 | 0.15 | 0.15 | 0.15 | 0.15 |
| Sodium Chloride | Sodium Chloride | 1 | 1 | 1 | 1 | 1 | 1 |
| | Total | 100 | 100 | 100 | 100 | 100 | 100 |

| | | | | | | | |
|---|---|---|---|---|---|---|---|
| 20 -Pureact W conc' commercially available from Innospec Performance Chemicals. | | | | | | | |

**Table 11**

| **Trade Name** | **Component** | **Formulation** | | | | | |
|---|---|---|---|---|---|---|---|
| | | **AE** | **AF** | **AG** | **AH** | **AI** | **AJ** |
| | Deionized Water | QS to 100% | QS to 100% | QS to 100% | QS to 100% | QS to 100% | QS to 100% |

| **Surfactants** | | | | | | | |
|---|---|---|---|---|---|---|---|
| Hostapon® SC185C | Sodium Cocoyl Isethionate | 2 | 2 | 2 | 2 | 2 | 2 |
| Iselux® flakes | Sodium laurovl Methyl | 3.5 | 3.5 | 3.5 | 3.5 | 3.5 | 3.5 |
| Tego® Betain F KH | Cocamidopropyl Betaine | 2.7 | 2.7 | 2.7 | 2.7 | 2.7 | 2.7 |
| Pureact W conc | Sodium Methyl Cocoyl Taurate | 1.5 | 1.5 | 1.5 | 1.5 | 1.5 | 1.5 |

| **Rheology Modifiers** | | | | | | | |
|---|---|---|---|---|---|---|---|
| Benecel™ E10M | Hydroxypropyl Methylcellulose | 0.5 | 0.5 | 0.5 | 0.5 | 0.5 | 0.5 |

| **Conditioners** | | | | | | | |
|---|---|---|---|---|---|---|---|
| N-Hance™ 4572 | Guar hydroxypropyltrimonium Chloride (and) Acrylamidopropyltrimonium Chloride/Acrylamide Copolymer | 0.15 | -- | -- | -- | 0.15 | -- |
| N-Hance™ 3215 | Guar hydroxypropyltrimonium | -- | 0.2 | -- | -- | -- | -- |
| N-Hance™ CG 13 | Guar hydroxypropyltrimonium | -- | -- | 0.2 | -- | -- | -- |
| N-Hance™ CCG45 | Guar hydroxypropyltrimonium | -- | -- | - | 0.2 | -- | -- |
| N-Hance™ 5182D | Acrylamidopropyltrimonium | -- | -- | -- | -- | -- | 0.2 |

| **Others** | | | | | | | |
|---|---|---|---|---|---|---|---|
| Dow Corning® 1788 | Silicone | -- | -- | -- | -- | 3 | 0 |
| Tegin® G 1100 | Glycol Distearate | 1.5 | 1.5 | 1.5 | 1.5 | 1.5 | 1.5 |
| Firmenich | Parfum | 0.3 | 0.3 | 0.3 | 0.3 | 0.3 | 0.3 |
| Optiphen™ MIT Ultra | Methylisothiazolinone / Phenylpropanol / Propylene Glycol | 0.3 | 0.3 | 0.3 | 0.3 | 0.3 | 0.3 |
| Sodium Hydroxide | Sodium Hydroxide (33%) | 0.01 | 0.01 | 0.01 | 0.01 | 0.01 | 0.01 |
| Dissolvine® Na | Disodium EDTA | 0.15 | 0.15 | 0.15 | 0.15 | 0.15 | 0.15 |
| Sodium Chloride | Sodium Chloride | 1 | 1 | 1 | 1 | 1 | 1 |
| | Total | 100 | 100 | 100 | 100 | 100 | 100 |

### Example 2 - Coacervate Formation Testing

The depositing performances of basic shampoos containing a cationic polymer can be predicted by the depth and width of the coacervate curve. The coacervate curve can be created by plotting the % transmittance at 600 nm of a shampoo at different dilution ranges. Formulations A-F, I, M, T, V and the Commercial Formulation were subjected to such testing for coacervate formation. Transparency % was measured at various dilution rates (defined as volume of water: volume of formulation) using an Agilent Cary 60 UV-vis spectrophotometer:

The formulations were each diluted with water to form samples having wt(formulation)/wt(water) ratios of 1:1, 1:2.5, 1:5, 1:7.5, 1:10, 1:12.5, 1:15, 1:17.5, 1:20, and 1:22.5 and individually placed in curvettes for testing. Each sample was individually tested in the spectrophotometer against a cuvette filled with water by measuring the transmittance at 600 nm wavelength light.

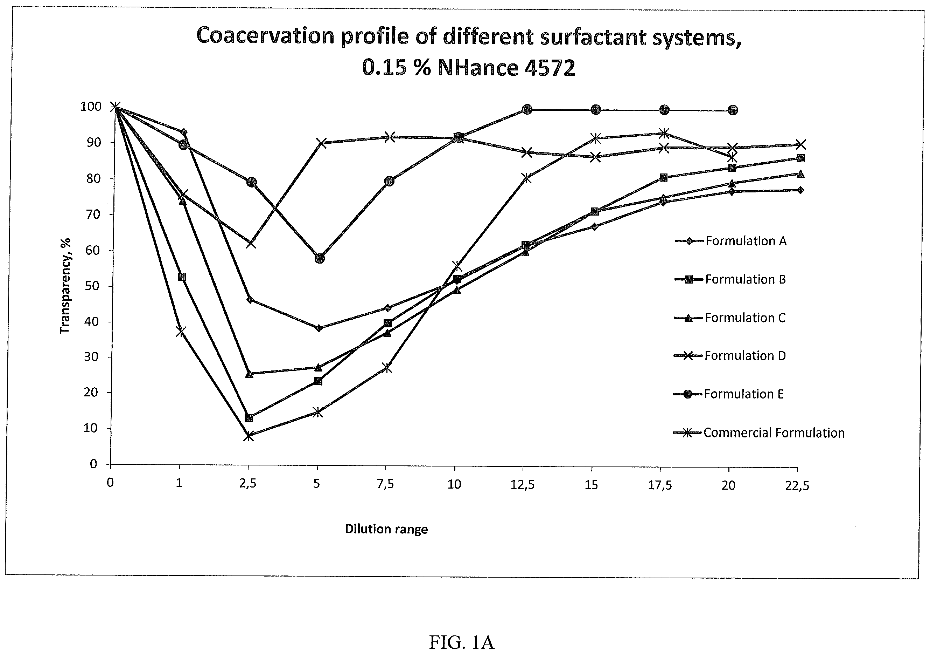

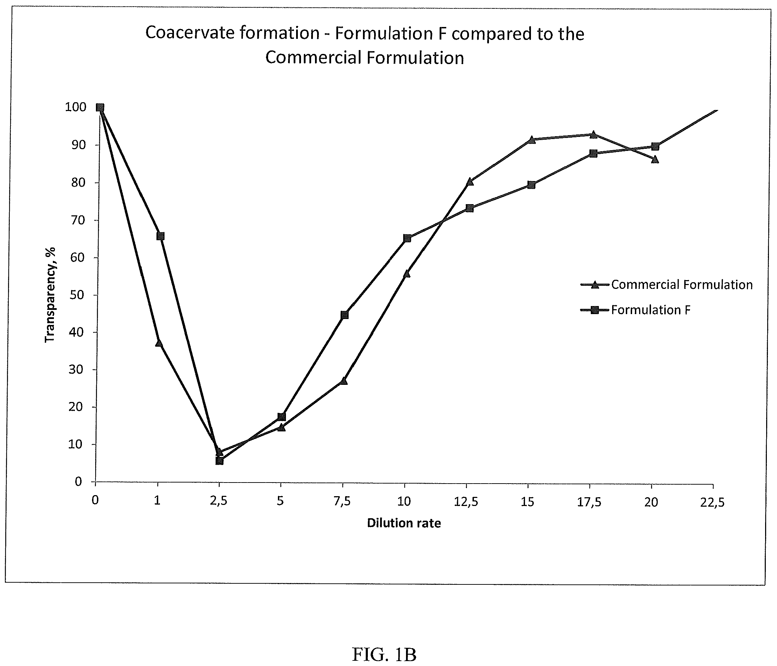

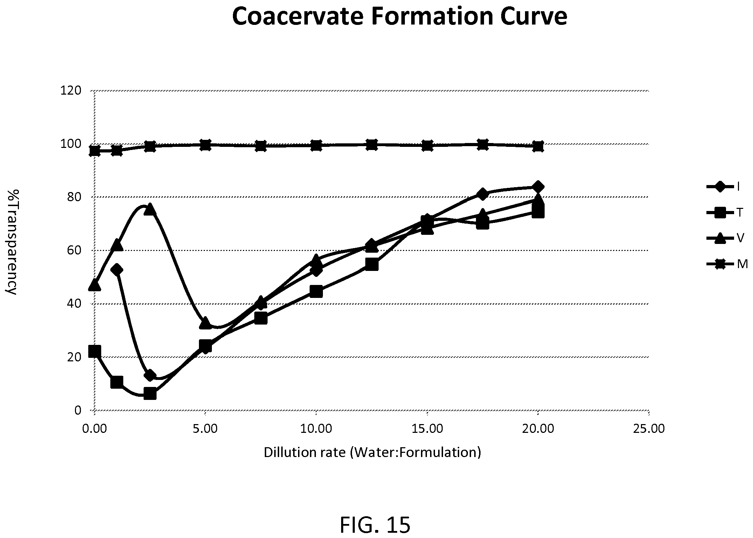

FIG. 1A shows the results of such testing for the Commercial Formulation and Formulations A-E. As can be seen in FIG. 1A, Formulations B and C show similar coacervate formation as compared to the Commercial Formulation, and Formulation A demonstrated effective coacervate formation. FIG. 1B shows the results of such testing for the Commercial Formulation and Formulation F. As can be seen in FIG. 1B, Formulation F shows comparable coacervate formation as compared to the Commercial Formulation. FIG. 15 shows the results of such testing for Formulations I, M, T and V. Formulations I and T show the minimum transmissions of 13.5% (Formulation I) and 6.5% (Formulation T).

### Example 3 - Combability Testing

### Wet Combability Testing

Bleached Caucasian human hair was separated into individual hair tresses weighing about 3 grams each. For each of the tests, the tress was rinsed with water and 0.2 g of the formulation per gram of tress was lathered into the hair tress for thirty seconds by stroking the tress downwardly. The tress was then rinsed for thirty seconds with water and 0.2 g of the formulation per gram of tress was applied to the tress for a second time, and lathered for thirty seconds by stroking the tress downwardly. The tress was then rinsed again for thirty seconds with water and excess water was removed by passing the tress between the index and middle fingers.

The wet comb total energy (gf-mm) was then measured using the Instron Wet Combing procedure. According to the Instron Wet Combing procedure, each hair tress was soaked for 15 minutes in distilled water. Excess water was removed by passing the tress through the index and middle fingers. The tress was untangled by combing the tress by hand. The tress was then dipped in distilled water three times to retangle the tress. Excess water was then removed by again passing the tress through the index and middle fingers. The tress was placed on a hanger and combed with the INSTRON instrument which uses an Instron strain gauge equipped to measure the total force required to comb the wet hair. Performance is evaluated by the ability of a particular formulation to reduce the required force. Each of the formulations was tested 3 separate times.

### Dry Combability Testing

Bleached Caucasian human hair was separated into individual hair tresses weighing about 3 grams each. For each of the tests, the tress was rinsed with water and 0.2 g of the formulation per gram of tress was lathered into the hair tress for thirty seconds by stroking the tress downwardly. The tress was then rinsed for thirty seconds with water and 0.2 g of the formulation per gram of tress was applied to the tress for a second time, and lathered for thirty seconds by stroking the tress downwardly. The tress was then rinsed again for thirty seconds with water and excess water was removed by passing the tress between the index and middle fingers.

The dry comb total energy (gf-mm) was then measured using the Instron Dry Combing procedure. According to the Instron Dry Combing procedure, each hair tress was soaked for 15 minutes in distilled water. Excess water was removed by passing the tress through the index and middle fingers. The tress was untangled by combing the tress by hand. The tress was then dipped in distilled water three times to retangle the tress. Excess water was then removed by again passing the tress through the index and middle fingers. The tress was then blow dried to remove any remaining water. The dried tress was placed on a hanger and combed with the INSTRON instrument which uses an Instron strain gauge equipped to measure the total force required to comb the dry hair. Performance is evaluated by the ability of a particular formulation to reduce the required force. Each of the formulations was tested 3 separate times.

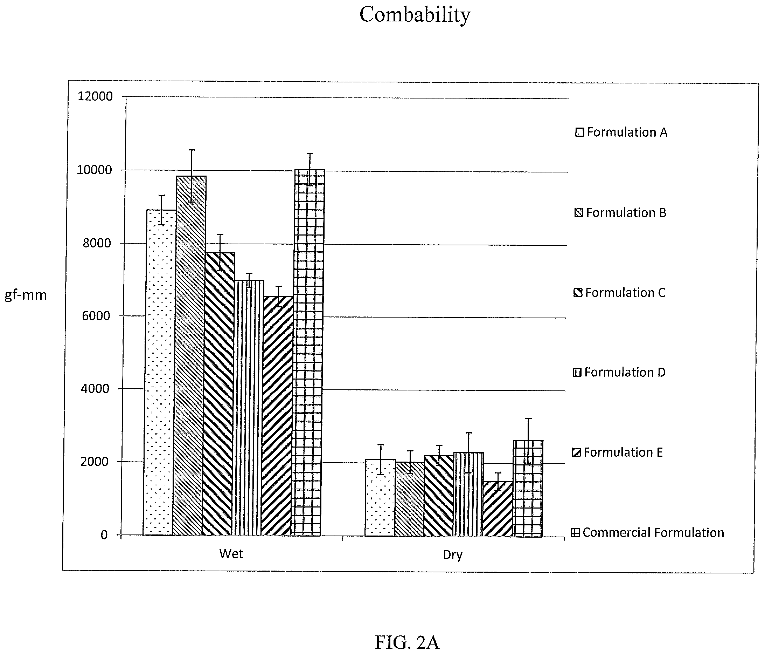

FIG. 2A shows the averages from the results of such testing for the Commercial Formulation and Formulations A-E. The data in FIG. 2A show that the wet and dry comb performance of Formulations A - E were each comparable to that of the Commercial Formulation.

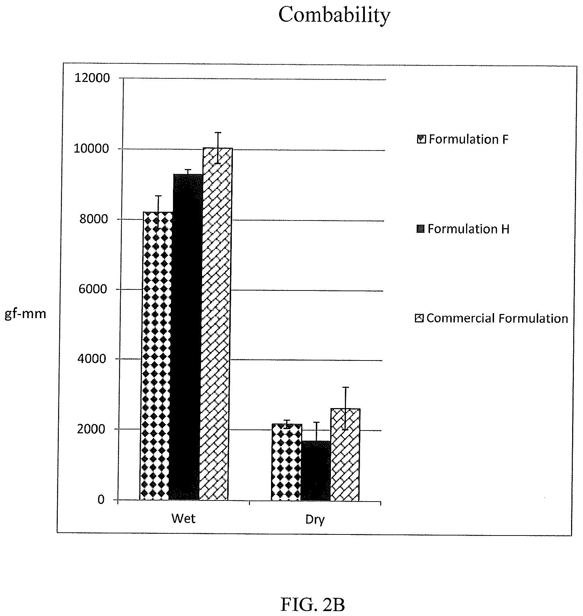

FIG. 2B shows the averages from the results of such testing for the Commercial Formulation and Formulations F and H. The data in FIG. 2B show that the wet and dry comb performance of Formulations F and H were comparable to that of the Commercial Formulation.

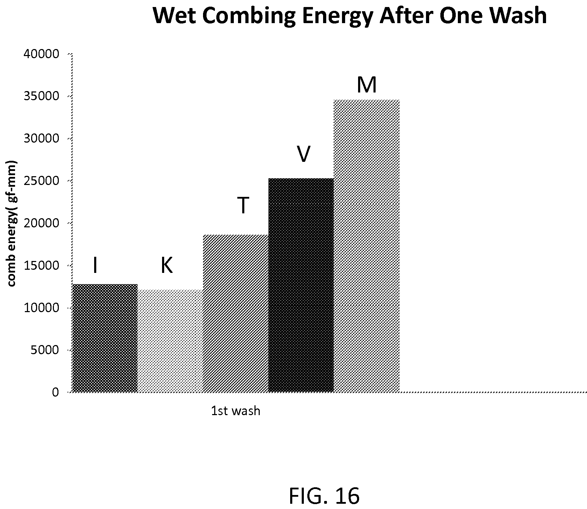

FIG. 16 shows the averages from the results of wet combing energy after one wash for Formulations I. K. M, T and V. Formulations I and K show the low web combing energy while Formulations T and V show combing improvement.

### Example 4 - Foam Stability Testing

The Commercial Formulation and Formulations B, F, I, J, K, L, M, T and V were subjected to foam stability testing using a Krüss Foam Analyzer DFA 100 instrument. The DFA 100 instrument enables the foaming of liquids and measurement of the initial form height and decay of the foam column over time. Samples of the formulations were diluted to 10 wt% with deionized water and then tested in the DFA 100 Analyzer at a temperature of 25°C, a stirring time of 15 sec, a measurement time of 616 sec, and with stirring at 4000 rpm.

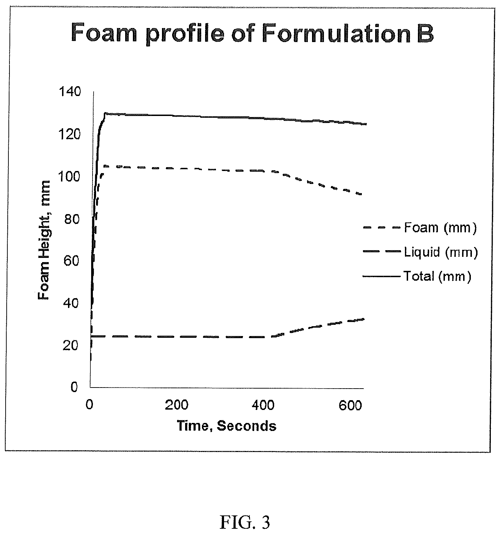

Results for testing of Formulation B are shown in FIG. 3. As can be seen in FIG. 3 Formulation B provides acceptable flash foam and foam stability.

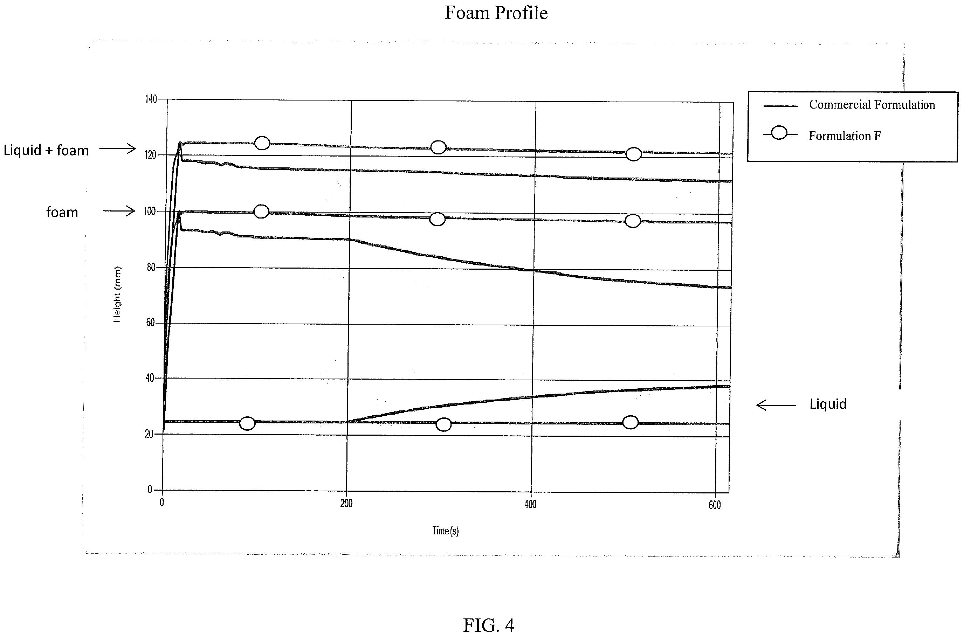

Results for testing of the Commercial Formulation and Formulation F are shown in FIG. 4. As can be seen in FIG. 4, Formulation F provides higher flash foam formation, and the foam starts to collapse much later than that for the Commercial Formulation.

Pictures were then taken of the foam produced for Formulation F and the Commercial Formulation after 3 minutes to evaluate bubble size. FIG.s 5 and 6 show the bubble sizes for Formulation F and the Commercial Formulation, respectively. As can be seen from FIG.s 5 and 6, Formulation F shows much smaller bubble sizes as compared to the Commercial Formulation, which is indicitave of a finer foam and increased foam stability.

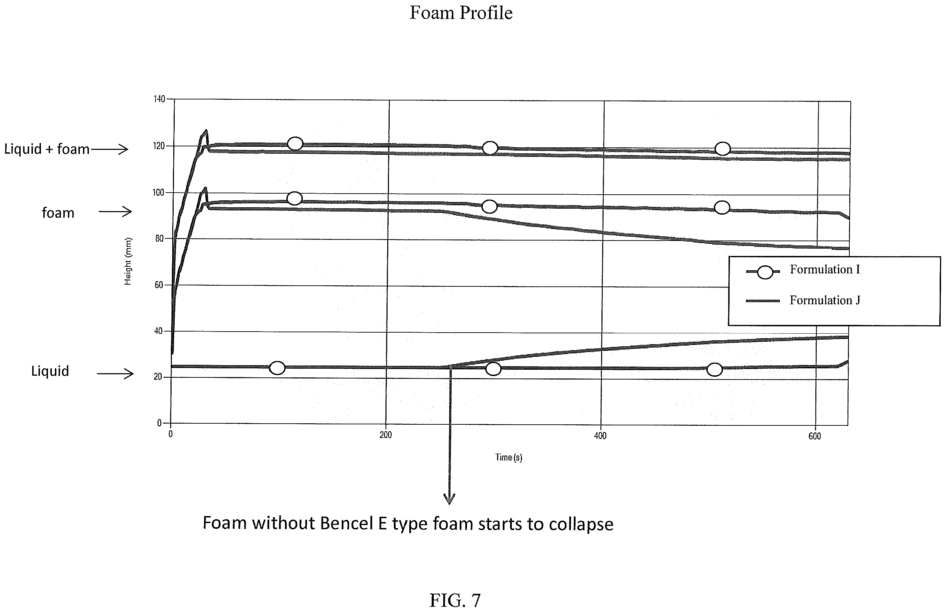

Results for testing of Formulations I and J are shown in FIG. 7. As can be seen in FIG. 7, Formulation I provides slightly higher flash foam formation and better foam stability than that for Formulation J (without Benecel™ E type rheology modifier).

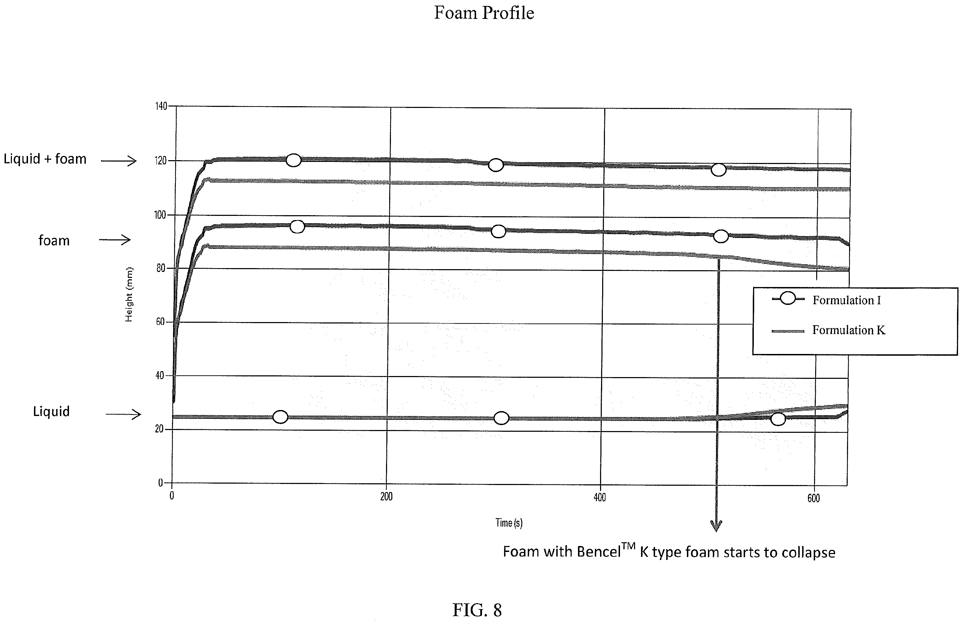

Results for testing of Formulations I and K are shown in FIG. 8. As can be seen in FIG. 8, Formulation I provides higher flash foam formation and slightly better foam stability than that for Formulation K (with Benecel™ K type rheology modifier rather than Benecel™ E type).

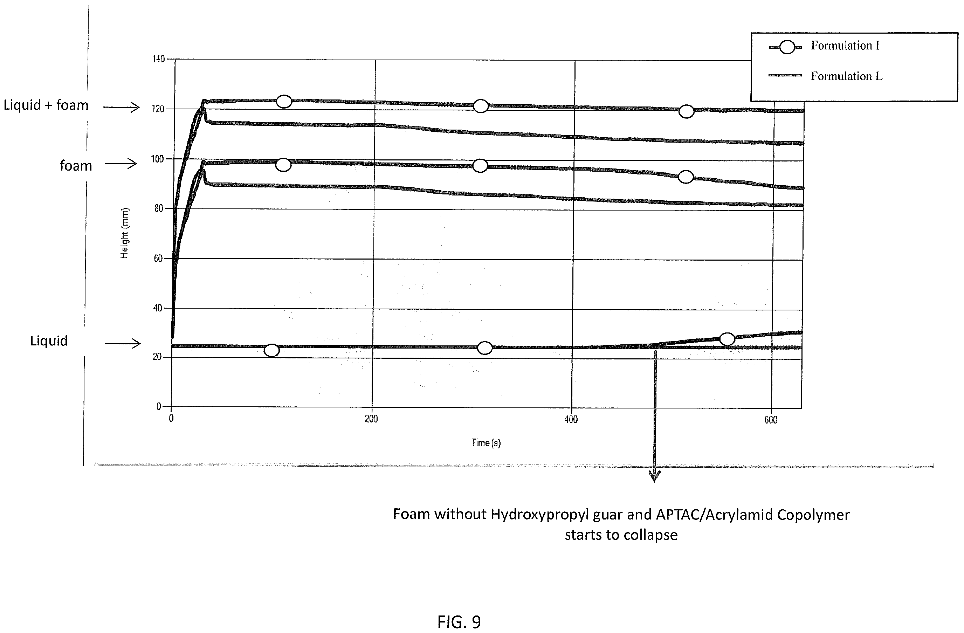

Results for testing of Formulations I and L are shown in FIG. 9. As can be seen in FIG. 9, Formulation I provides higher flash foam formation and slightly better foam stability than that for Formulation L (without hydroxypropyl guar and APTAC/acrylamide copolymer).

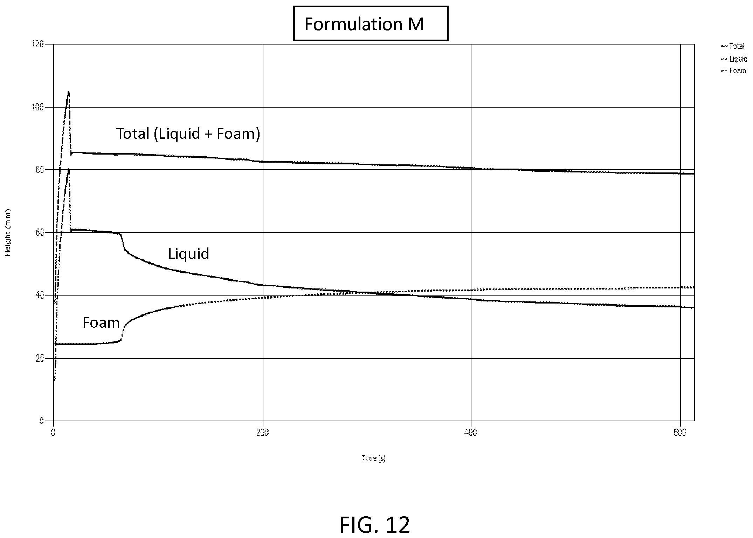

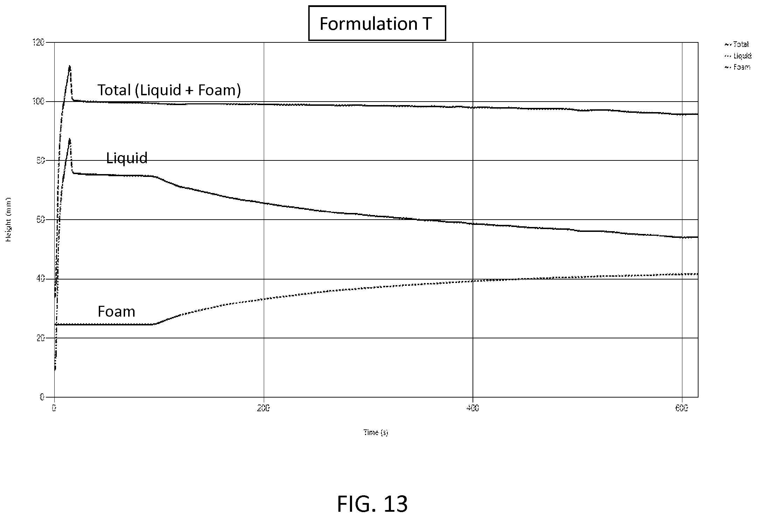

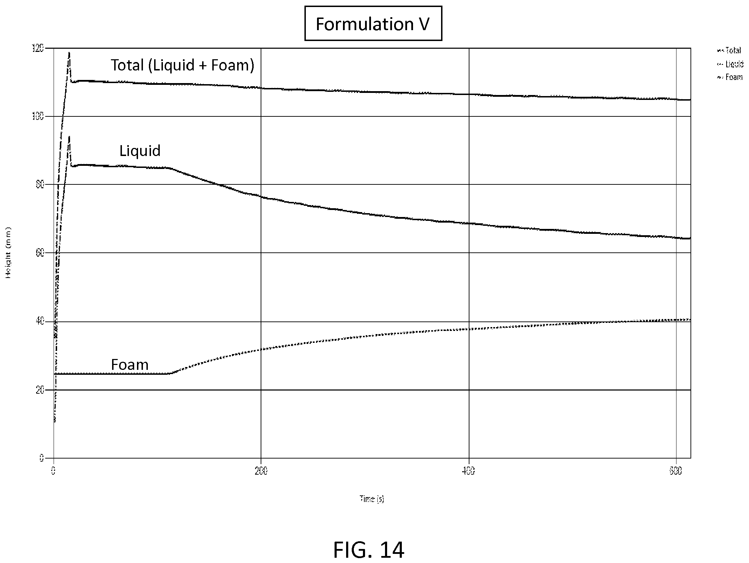

Results for testing of Formulations M, T and V are shown in FIG. 12, FIG. 13 and FIG. 14. Formulation V provides stable foam.

### Example 5 - Sensory Evaluation Testing

Formulation B and the Commercial Formulation were separately applied to hair tresses, as described above in Example 3, and the hair tresses were subjected to sensory testing.

For wet state performance, the tresses were tested for: detangling, combability, stickiness, slipperiness, smoothness, and coatedness.

The wet state performance testing was performed in accordance with the following method:
- Use bleached hair,
- Two tresses used per sample,
- Maximum 10 tresses per time (i.e. 5 samples per time),
- Clean the hair tresses with 4.5% sodium lauryl sulfate (SLS) solution,
- Shampoo the tresses as described above (i.e. 0.1g shampoo /g hair or other treatment),
- During shampooing (treatment), sensory evaluate the foam speed, foam structure (big bubble, creamy foam, etc..), the amount of foam and the feel during kneading,
- Rinse for 30 seconds with water at 37°C, and sensory evaluate the foam speed, foam structure (big bubble, creamy foam, etc..), the amount of foam and the feel during kneading,
- After rinse-off, sensory evaluate for wet feel (including: stickiness, smoothness, slipperiness, coatedness), wet combability, and detangling,
- Place the hair tresses in the humidity chamber, at the following condition: T= 23°C, RH= 50% to dry overnight.

For dry state performance, the dry tresses were further tested for: sheen, detangling, combability, fly away, volume, slipperiness, smoothness, coatedness, and dryness.

The evaluations were performed by six trained panelists (the evaluation has to be performed by five persons, minimum).

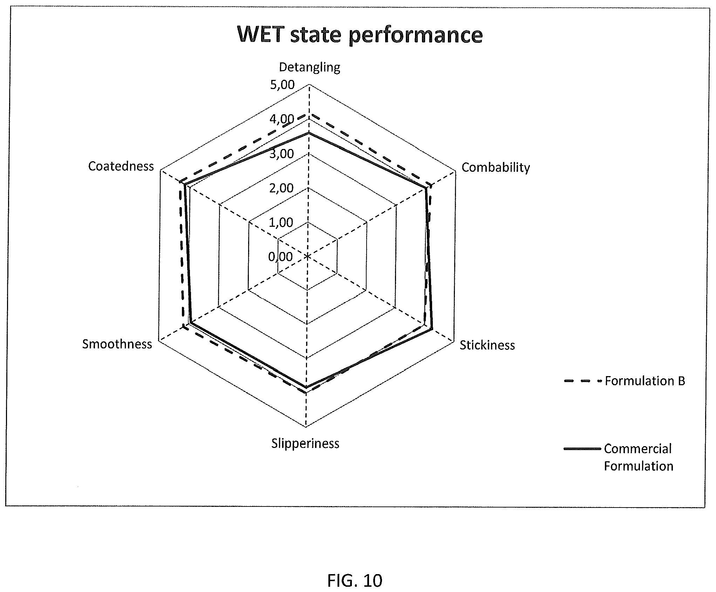

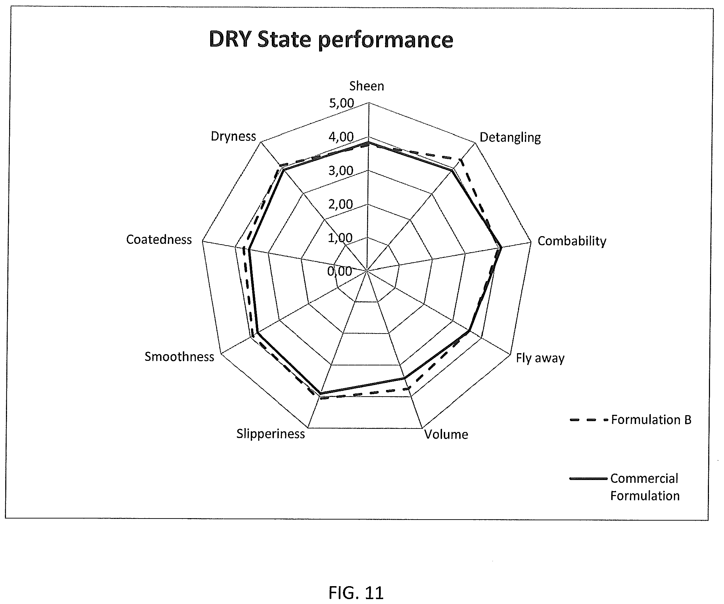

The results of the wet state performance testing are shown in FIG. 10, and shows that Formulation B demonstrated equal or slightly better performance in wet sensory testing as compared to the Commercial Formulation. The results of the dry state performance testing are shown in FIG. 11, and shows that Formulation B demonstrated equal or slightly better performance in dry sensory testing as compared to the Commercial Formulation.

## Claims

1. A personal care cleansing composition comprising:
a) water;
b) up to 10 wt%, based on the total weight of the personal care cleansing composition, of a surfactant selected from the group consisting of an anionic surfactant, an amphoteric surfactant, a nonionic/anionic surfactant mixture, and combinations thereof;
c) a rheology modifying polymer selected from the group consisting of carboxymethylcellulose, hydroxyethylcellulose, hydroxypropylcellulose, hydroxypropylmethylcellulose, hydroxypropyl-Guar, hydroxymethylhydroxy-ethylcellulose, and combinations thereof; which is present in an amount ranging from 0.1 to 1.5 wt%, based on the total weight of the personal care cleansing composition;
d) a cationic-substituted guar; and
e) a copolymer of acrylamidopropyltrimonium chloride and acrylamide,
wherein the personal care cleansing composition is sulfate-free.

2. The personal care cleansing composition of claim 1, wherein the anionic surfactant comprises a compound selected from the group consisting of an ammonium, alkali or alkali earth salt of: a sulfonate, a sulfosuccinate, a carboxylate, a sarcosinate, an isethionate, a sulfoacetate and combinations thereof;
preferably, wherein the anionic surfactant comprises a compound selected from the group consisting of sodium alpha-olefin sulfonate, disodium laureth sulfosuccinate, sodium laureth-5 (13) carboxylate, sodium lauroyl sarcosinate, sodium cocoyl isethionate, sodium lauryl sulfoacetate, and combinations thereof.

3. The personal care cleansing composition of claim 1, wherein the amphoteric surfactant comprises a compound selected from the group consisting of coco amido propyl betaine, cocoamido hydroxyl sultaine, cocamphoacetate, sodium methyl cocoyl taurate, and combinations thereof.

4. The personal care cleansing composition of claim 1, wherein the nonionic surfactant comprises a compound selected from the group consisting of an alkyl glucoside, cocoamide monoethanolamine, cocoamide diethanolamine, a glycerol alkyl ester, polyethylene glycol, and combinations thereof.

5. The personal care cleansing composition of claim 1, wherein the rheology modifying polymer comprises hydroxypropylmethylcellulose having a methoxyl content between 26 and 32 wt%, a hydroxypropyl content between 6 and 12 wt%, and a viscosity between 40 and 16000 mPas.

6. The personal care cleansing composition of claim 1, wherein the cationic-substituted guar
- has a degree of cationic substitution of 0.1 to 0.4, and an average molecular weight from 800,000 to 1,800,000 Dalton; or
- is guar hydroxypropyltrimonium chloride.

7. The personal care cleansing composition of claim 1, wherein the cationic-substituted guar is a guar substituted with at least one cationic moiety selected from compounds having the formula: AB; wherein
- A, independently, is selected from a linear or branched, substituted or unsubstituted C₁ - C₆ alkyl radical;
- B, independently, is selected from S⁺R₁R₂X⁻, N⁺R₁R₂R₃X⁻, P⁺R₁R₂R₃X⁻, wherein R₁, R₂, and R₃, independently, are selected from the group consisting of hydrogen and linear and branched C₁ - C₂₄ alkyl, and X⁻ is an anion;

8. The personal care cleansing composition of claim 7, wherein
- A comprises a compound selected from the group consisting of 3-halo-2-hydroxypropyl group; 2,3-epoxy propyl group; and combinations thereof; or
- the at least one cationic moiety is substituted on a hydroxy group of the guar.

9. The personal care cleansing composition of claim 1, wherein the copolymer has a charge density of 0.75 to 3.0, and an average molecular weight from 1,000,000 to 2,000,000 Dalton.

10. The personal care cleansing composition of claim 1, wherein the cationic-substituted guar is present in an amount ranging from 0.05 to 1.5 wt%, based on the total weight of the personal care cleansing composition.

11. The personal care cleansing composition of claim 1, wherein the copolymer is present in an amount ranging from 0.01 to 0.25 wt%, based on the total weight of the personal care cleansing composition.

12. The personal care cleansing composition of claim 1 further comprising a metal halide, wherein the personal care cleansing composition has a higher viscosity as compared to an identical composition not including such metal halide.

13. The personal care cleansing composition of claim 1 having a % transparency
- of less than 50%; or
- of less than 30%
at a water dilution ranging from 2.5 to 5 (volume water: volume personal care cleansing composition), as measured by a spectrophotometer.

14. The personal care cleansing composition of claim 1 having a foam height
- of at least 85 mm after 300 seconds; or
- of at least 80 mm after 400 seconds.

## Patentansprüche

1. Eine Körperpflegereinigungszusammensetzung umfassend:
a) Wasser;
b) bis zu 10 Gew.-%, bezogen auf das Gesamtgewicht der Körperpflegereinigungszusammensetzung, eines Tensids ausgewählt aus der Gruppe bestehend aus einem anionischen Tensid, einem amphoteren Tensid, einer Mischung aus nicht-ionischem/anionischem Tensid und deren Kombinationen;
c) ein rheologiemodifizierendes Polymer ausgewählt aus der Gruppe bestehend aus Carboxymethylcellulose, Hydroxyethylcellulose, Hydroxypropylcellulose, Hydroxypropylmethylcellulose, Hydroxypropyl-Guar, Hydroxymethylhydroxyethylcellulose und deren Kombinationen, das in einer Menge im Bereich von 0,1 bis 1,5 Gew.-% vorhanden ist, bezogen auf das Gesamtgewicht der Körperpflegereinigungszusammensetzung;
d) ein kationisch substituiertes Guar; und
e) ein Copolymer aus Acrylamidopropyltrimoniumchlorid und Acrylamid,
wobei die Körperpflegereinigungszusammensetzung sulfatfrei ist.

2. Die Körperpflegereinigungszusammensetzung gemäß Anspruch 1, wobei das anionische Tensid eine Verbindung umfasst, die ausgewählt wird aus der Gruppe bestehend aus einem Ammonium-, Alkali- oder Erdalkalisalz von einem Sulfonat, einem Sulfosuccinat, einem Carboxylat, einem Sarcosinat, einem Isethionat, einem Sulfoacetat und deren Kombinationen;
bevorzugt wobei das anionische Tensid eine Verbindung umfasst, die ausgewählt wird aus der Gruppe bestehend aus Natrium-alpha-olefinsulfonat, Dinatriumlaurethsulfosuccinat, Natriumlaureth-5 (13)carboxylat, Natriumlauroylsarcosinat, Natriumcocoylisethionat, Natriumlaurylsulfoacetat und deren Kombinationen.

3. Die Körperpflegereinigungszusammensetzung gemäß Anspruch 1, wobei das amphotere Tensid eine Verbindung umfasst ausgewählt aus der Gruppe bestehend aus Cocoamidopropylbetain, Cocoamidohydroxylsultain, Cocoamphoacetat, Natriummethylcocoyltaurat und deren Kombinationen.

4. Die Körperpflegereinigungszusammensetzung gemäß Anspruch 1, wobei das nichtionische Tensid eine Verbindung umfasst ausgewählt aus der Gruppe bestehend aus Alkylglucosid, Cocoamidmonoethanolamin, Cocoamiddiethanolamin, einem Glycerolalkylester, Polyethylenglycol und deren Kombinationen.

5. Die Körperpflegereinigungszusammensetzung gemäß Anspruch 1, wobei das rheologiemodifizierende Polymer Hydroxypropylmethylcellulose mit einem Methoxylgehalt zwischen 26 und 32 Gew.-%, einem Hydroxypropylgehalt zwischen 6 und 12 Gew.-% und einer Viskosität zwischen 40 und 16000 mPas umfasst.

6. Die Körperpflegereinigungszusammensetzung gemäß Anspruch 1, wobei das kationisch substituierte Guar
- einen Grad der kationischen Substitution von 0,1 bis 0,4 und ein durchschnittliches Molekulargewicht von 800 000 bis 1 800 000 Dalton aufweist; oder
- Guar-Hydroxypropyltrimoniumchlorid ist.

7. Die Körperpflegereinigungszusammensetzung gemäß Anspruch 1, wobei das kationisch substituierte Guar ein Guar ist, das mit mindestens einer kationischen Einheit substituiert ist, die aus Verbindungen mit der Formel: AB ausgewählt ist; wobei
- A unabhängig voneinander aus einem linearen oder verzweigten, substituierten oder unsubstituierten C₁ - C₆-Alkylrest ausgewählt wird;
- B unabhängig voneinander ausgewählt wird aus S⁺R₁R₂X⁻, N⁺R₁R₂R₃X⁻, P⁺R₁R₂R₃X⁻, wobei R₁, R₂ und R₃ unabhängig voneinander aus der Gruppe bestehend aus Wasserstoff und linearem und verzweigtem C₁ - C₂₄ Alkyl ausgewählt werden und X⁻ ein Anion ist.

8. Die Körperpflegereinigungszusammensetzung gemäß Anspruch 7, wobei
- A eine Verbindung umfasst, die ausgewählt wird aus der Gruppe bestehend aus 3-Halogen-2-hydroxypropylgruppe, einer 2,3-Epoxypropylgruppe und deren Kombinationen; oder
- die mindestens eine kationische Einheit an einer Hydroxygruppe des Guar substituiert ist.

9. Die Körperpflegereinigungszusammensetzung gemäß Anspruch 1, wobei das Copolymer eine Ladungsdichte von 0,75 bis 3,0 und ein durchschnittliches Molekulargewicht von 1 000 000 bis 2 000 000 Dalton aufweist.

10. Die Körperpflegereinigungszusammensetzung gemäß Anspruch 1, wobei das kationisch substituierte Guar in einer Menge im Bereich von 0,05 bis 1,5 Gew.-%, bezogen auf das Gesamtgewicht der Körperpflegereinigungszusammensetzung, vorhanden ist.

11. Die Körperpflegereinigungszusammensetzung gemäß Anspruch 1, wobei das Copolymer in einer Menge von 0,01 bis 0,25 Gew.-%, bezogen auf das Gesamtgewicht der Körperpflegereinigungszusammensetzung, vorhanden ist.

12. Die Körperpflegereinigungszusammensetzung gemäß Anspruch 1, die weiterhin ein Metallhalogenid umfasst, wobei die Körperpflegereinigungszusammensetzung im Vergleich zu einer identischen Zusammensetzung, die ein solches Metallhalogenid nicht enthält, eine höhere Viskosität aufweist.

13. Die Körperpflegereinigungszusammensetzung gemäß Anspruch 1, die eine Transparenz in %
- von weniger als 50% oder
- von weniger als 30%
bei einer Wasserverdünnung von 2,5 bis 5 (Volumen Wasser: Volumen Körperpflegereinigungszusammensetzung), gemessen mit einem Spektrometer, aufweist.

14. Die Körperpflegereinigungszusammensetzung gemäß Anspruch 1, die eine Schaumhöhe
- von mindestens 85 mm nach 300 Sekunden; oder
- von mindestens 80 mm nach 400 Sekunden aufweist.

## Revendications

1. Composition nettoyante de soin personnel comprenant :
a) de l'eau ;
b) jusqu'à 10 % en poids, sur la base du poids total de la composition nettoyante de soin personnel, d'un tensioactif choisi dans le groupe constitué d'un tensioactif anionique, d'un tensioactif amphotère, d'un mélange de tensioactif non ionique/anionique, et des combinaisons de ceux-ci ;
c) un polymère modifiant la rhéologie choisi dans le groupe constitué des carboxyméthylcellulose, hydroxyéthylcellulose, hydroxypropylcellulose, hydroxypropylméthylcellulose, hydroxypropyl-guar, hydroxyméthylhydroxy-éthylcellulose, et des combinaisons de ceux-ci ; qui est présent en une quantité dans la plage de 0,1 à 1,5 % en poids, sur la base du poids total de la composition nettoyante de soin personnel ;
d) un guar à substitution cationique ; et
e) un copolymère de chlorure d'acrylamidopropyltrimonium et d'acrylamide,
la composition nettoyante de soin personnel étant exempte de sulfate.

2. Composition nettoyante de soin personnel selon la revendication 1, dans laquelle le tensioactif anionique comprend un composé choisi dans le groupe constitué d'un sel d'ammonium, de métal alcalin ou de métal alcalino-terreux de : un sulfonate, un sulfosuccinate, un carboxylate, un sarcosinate, un iséthionate, un sulfoacétate et des combinaisons de ceux-ci ;
de préférence, dans laquelle le tensioactif anionique comprend un composé choisi dans le groupe constitué d'un alpha-oléfine-sulfonate de sodium, lauréth-sulfosuccinate disodique, lauréth-5(13)carboxylate de sodium, lauroyl-sarcosinate de sodium, cocoyl-iséthionate de sodium, lauryl-sulfoacétate de sodium, et des combinaisons de ceux-ci.

3. Composition nettoyante de soin personnel selon la revendication 1, dans laquelle le tensioactif amphotère comprend un composé choisi dans le groupe constitué des cocoamidopropylbétaïne, cocoamidohydroxylsultaïne, cocamphoacétate, méthylcocoyl-taurate de sodium, et des combinaisons de ceux-ci.

4. Composition nettoyante de soin personnel selon la revendication 1, dans laquelle le tensioactif non ionique comprend un composé choisi dans le groupe constitué d'un alkylglucoside, cocoamide-monoéthanolamine, cocoamide-diéthanolamine, ester d'alkyle de glycérol, polyéthylène glycol, et des combinaisons de ceux-ci.

5. Composition nettoyante de soin personnel selon la revendication 1, dans laquelle le polymère modifiant la rhéologie comprend de l'hydroxypropylméthylcellulose ayant une teneur en méthoxyle comprise entre 26 et 32 % en poids, une teneur en hydroxypropyle comprise entre 6 et 12 % en poids, et une viscosité comprise entre 40 et 16 000 mPas.

6. Composition nettoyante de soin personnel selon la revendication 1, dans laquelle le guar à substitution cationique
- a un degré de substitution cationique de 0,1 à 0,4, et un poids moléculaire moyen de 800 000 à 1 800 000 daltons ; ou
- est le chlorure de guar-hydroxypropyltrimonium.

7. Composition nettoyante de soin personnel selon la revendication 1, dans laquelle le guar à substitution cationique est un guar substitué par au moins un fragment cationique choisi parmi des composés ayant la formule : AB ; dans laquelle
- A est indépendamment choisi parmi un radical alkyle en C₂-C₆ linéaire ou ramifié, substitué ou non substitué ;
- B est indépendamment choisi parmi S⁺R₁R₂X⁻, N⁺R₁R₂R₃X⁻, P⁺R₁R₂R₃X⁻, où R₁, R₂ et R₃, sont indépendamment choisis dans le groupe constitué d'hydrogène et alkyle en C₁-C₂₄ linéaire et ramifié, et X⁻ est un anion.

8. Composition nettoyante de soin personnel selon la revendication 7, dans laquelle
- A comprend un composé choisi dans le groupe constitué d'un groupe 3-halogéno-2-hydroxy-propyle ; un groupe 2,3-époxypropyle ; et des combinaisons de ceux-ci ; ou
- l'au moins un fragment cationique est substitué sur un groupe hydroxy du guar.

9. Composition nettoyante de soin personnel selon la revendication 1, dans laquelle le copolymère a une densité de charge de 0,75 à 3,0, et un poids moléculaire moyen de 1 000 000 à 2 000 000 daltons.

10. Composition nettoyante de soin personnel selon la revendication 1, dans laquelle le guar à substitution cationique est présent en une quantité dans la plage de 0,05 à 1,5 % en poids, sur la base du poids total de la composition nettoyante de soin personnel.

11. Composition nettoyante de soin personnel selon la revendication 1, dans laquelle le copolymère est présent en une quantité dans la plage de 0,01 à 0,25 % en poids, sur la base du poids total de la composition nettoyante de soin personnel.

12. Composition nettoyante de soin personnel selon la revendication 1 comprenant en outre un halogénure métallique, la composition nettoyante de soin personnel ayant une viscosité plus élevée qu'une composition identique ne comprenant pas un tel halogénure métallique.

13. Composition nettoyante de soin personnel selon la revendication 1 ayant un % de transparence
- inférieur à 50 % ; ou
- inférieur à 30 %
à une dilution dans l'eau dans la plage de 2,5 à 5 (volume d'eau : volume de composition nettoyante de soin personnel), tel que mesuré par un spectrophotomètre.

14. Composition nettoyante de soin personnel selon la revendication 1 ayant une hauteur de mousse
- d'au moins 85 mm après 300 secondes ; ou
- d'au moins 80 mm après 400 secondes.
